# DEMANDE DE BREVET EUROPEEN

(11) **EP 4 542 222 A1**
(43) Date de publication de la demande: **23.04.2025**
(21) Numéro de dépôt: 24206317.0
(22) Date de dépôt: 14.10.2024
(51) Int. Cl.: G01N 31/22, G01N 33/00, G01N 21/77, G01N 21/78

(54) **SYSTÈME COLORIMÉTRIQUE ET FLUOROGÉNIQUE POUR LA DÉTECTION D'UNE SUBSTANCE CHIMIQUE ET PROCÉDÉ DE DÉTECTION D'UNE SUBSTANCE CHIMIQUE LE METTANT EN OEUVRE**

(30) Priorité: 16.10.2023 FR 2311151
(71) Demandeur: Commissariat à l'Energie Atomique et aux Energies Alternatives, 75015 Paris (FR)
(72) Inventeur: TERMEAU, Lucile, 38054 Grenoble cedex 09 (FR); BUREAU, Valentin, 38054 Grenoble cedex 09 (FR); DE SOUSA NOBRE, Sonia, 38054 Grenoble cedex 09 (FR); PENLOU, Sébastien, 38054 Grenoble cedex 09 (FR)
(74) Mandataire: Brevalex

(57) **Abrégé**

L'invention concerne un système de détection d'une substance chimique, comprenant au moins un composé fluorescent à capacité de virage colorimétrique porté par des particules d'au moins deux différents types, disposées sur un support solide. Un procédé de détection d'une substance chimique dans un milieu comprend la mise en contact de ce milieu avec les particules portant le composé de ce système de détection, l'acquisition des réponses colorimétriques et des réponses fluorogéniques induites par cette mise en contact, et la déduction, en fonction de l'ensemble desdites réponses, de l'absence ou de la présence spécifique de ladite substance dans le milieu.

## Description

La présente invention s'inscrit dans le domaine de la détection et l'identification de substances chimiques susceptibles d'être contenues dans un milieu.

Plus particulièrement, la présente invention concerne un système de détection d'une ou plusieurs substances chimiques, ainsi qu'un procédé de préparation d'un tel système, et un kit de détection d'une ou plusieurs substances chimiques le comprenant. L'invention concerne également un procédé de détection d'une substance chimique susceptible d'être contenue dans un milieu, mettant en oeuvre un tel système.

L'invention trouve tout particulièrement et avantageusement application pour la détection, et le cas échéant l'identification, de substances chimiques toxiques, en particulier de substances organophosphorées interdites par l'Organisation pour l'interdiction des Armes Chimiques (OIAC), telles que le sarin, le tabun, le soman, le VX, les Novitchoks, ou encore de pesticides organophosphorés, tels que le malathion, le fenthion, le diphényl chlorophosphate (DPCP), le parathion ou le paraoxon-méthyl, de composés toxiques de guerre, notamment agents vésicants et agents vomitifs, tels que les composés arséniés, et de composés toxiques industriels (connus sous la dénomination TIC).

En particulier, les substances organophosphorées présentent une toxicité avérée pour l'organisme humain. En effet, ces substances peuvent être impliquées dans le mécanisme d'inhibition des sérineprotéases et, notamment, de l'acétylcholinestérase, qui intervient dans les jonctions synaptiques et dont le dérèglement de l'activité peut empêcher le relâchement musculaire et ainsi provoquer la mort par asphyxie.

Ces substances peuvent être comprises dans la formulation d'insecticides, de pesticides ou encore d'agents chimiques de combat (tels que les composés organophosphorés de la série G, comme le sarin (dit GB) ou les composés organophosphorés de la série V, comme le VX), et, du fait de la létalité élevée de ces substances, il s'avère important de pouvoir disposer de systèmes permettant leur détection et leur identification préliminaire.

Certains systèmes de détection utilisés à ce jour à cet effet sont basés sur des technologies impliquant des moyens de mesure physique, telles que la spectroscopie à mobilité ionique, la photométrie de flamme, les spectroscopies infrarouge et Raman, avec, pour difficultés, que ces systèmes nécessitent un appareillage complexe onéreux et pas forcément adapté à tous les milieux d'intervention en termes de masse et d'encombrement, auxquelles s'ajoute l'expertise de l'opérateur à considérer.

Il a autrement été proposé par l'art antérieur de détecter la présence d'une substance organophosphorée, en phase liquide, par deux techniques combinées, en particulier par voie colorimétrique et voie électrochimique (De et al., 2010, Tetrahedron Letters, 51, 1754-1757), ou par voie fluorogénique et voie électrochimique (Guo et al., 2011, Talanta, 87, 276- 283). Ces procédés de détection sont toutefois peu pratiques à réaliser, et ils ne peuvent être mis en oeuvre qu'en laboratoire, ne permettant pas une détection sur le terrain. Ils présentent en outre une faible précision de détection des substances cibles, et ne permettent pas de discriminer une substance chimique particulière d'autres substances de structure proche.

Le document FR 3130980 décrit un système de détection de composés chimiques à base de particules imprégnées d'indicateurs de détection, tels que des indicateurs colorés. Ce système comprend un premier type de particules qui est imprégné par un premier indicateur de détection, et un second type de particules qui est imprégné par un second indicateur de détection distinct du premier indicateur de détection.

Le document FR 3130978 décrit l'utilisation de composés spécifiques comme indicateurs de détection pour la détection de composés chimiques toxiques. Chacun de ces composés peut être porté par des particules d'un type donné. Au vu de ce qui existe, les présents inventeurs se sont orientés vers la conception d'un système de détection utilisable pour la détection d'une ou plusieurs substances chimiques, lequel système devant permettre la détection de ladite ou desdites substances chimiques et, lorsqu'il y en a plusieurs, idéalement, la discrimination desdites substances chimiques, et répondant à des critères de sensibilité, fiabilité, rapidité, portabilité, praticité d'utilisation, et qui puisse permettre une détection et, le cas échéant, une discrimination simple et, par exemple, visuelle à l'oeil nu, ou au moyen d'appareillage portable, avec une haute précision.

A cet effet, les présents inventeurs se sont tournés vers le développement d'un système de détection en phase solide, alliant praticité d'utilisation, de transport et de stockage. Ils ont découvert que des composés répondant à une formule générale particulière présentent des propriétés optiques, plus particulièrement des propriétés colorimétriques et des propriétés de fluorescence, qui sont modifiées par leur mise en contact avec des substances chimiques cibles, en particulier des substances organophosphorées. En outre, lorsque ces composés sont portés par des particules solides, leurs propriétés optiques sont également affectées en fonction du type particulier de particules mises en oeuvre, et, de manière particulièrement surprenante, leurs réponses colorimétrique et/ou fluorogénique provoquées par leur mise en contact avec une substance chimique, sont également affectées différemment selon le type de particules qui les porte. Chaque substance chimique cible provoque en effet une réponse colorimétrique (absence ou présence de virage colorimétrique, et caractéristiques de ce virage) et une réponse fluorogénique (absence ou présence de variation du pic de fluorescence, et caractéristiques de variation en termes d'intensité et/ou de longueur d'onde) qui sont spécifiques pour chaque couple de composé de détection et type de particules donné, et qui, pour un même composé, peuvent varier en fonction du type de particules qui lui est associé.

Ainsi, il a été découvert par les présents inventeurs que la mise en oeuvre d'un composé de formule générale particulière, porté respectivement par une pluralité de types de particules différents, et de la combinaison d'une lecture colorimétrique et d'une lecture de fluorescence, permet de détecter en phase solide, et avec une grande précision, et même discriminer, en choisissant correctement les couples « particules / composé » utilisés pour la détection, une substance chimique cible spécifique contenue dans un milieu. Cette détection, et le cas échéant identification, peut avantageusement être réalisée facilement et rapidement, de manière visuelle à l'œil nu, sous lumière du jour pour la lecture colorimétrique, et, pour la lecture de fluorescence, en faisant uniquement intervenir une lampe à ultraviolets (UV) émettant à une longueur d'onde appropriée, et/ou au moyen d'appareillages portables de terrain usuels, tel qu'un spectromètre, donnant accès à des informations quantifiables et des sources d'éclairage UV.

Ainsi, selon un premier aspect, il est proposé selon la présente invention un système de détection d'une ou plusieurs substances chimiques, en particulier sous forme liquide, susceptibles d'être contenues dans un milieu, ce système comprenant au moins un composé de formule générale (I) : 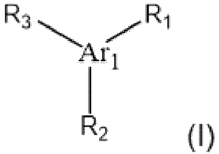 dans laquelle
Ar₁ représente un hétérocycle aromatique azoté, éventuellement substitué par un ou plusieurs radicaux alkyle linéaires ou ramifiés, de préférence en C1-C6, ou Ar₁ représente un groupe aromatique polycyclique condensé contenant au moins un hétérocycle aromatique azoté, chacun des cycles dudit groupe aromatique polycyclique condensé étant carbocyclique ou azoté, et étant éventuellement substitué par un ou plusieurs radicaux alkyle linéaires ou ramifiés, de préférence en C1-C6,
R₁, R₂ et R₃, identiques ou différents, représentent chacun un atome d'hydrogène ou un groupe R₄ de formule générale (II) : 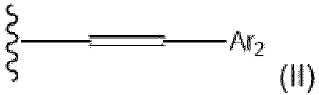 dans laquelle
   Ar₂ représente un groupe phényl substitué par un groupement Rs choisi parmi un atome d'hydrogène, un atome d'halogène, en particulier de brome, un groupe amino primaire -NH₂, un groupe hydroxyle -OH, un groupe sulfhydryle -SH et un groupement -R₆, -NHR₆, -N(R₆)₂, -OR₆ ou -SR₆, où R₆ représente un radical alkyle linéaire ou ramifié, de préférence en C1-C20, notamment méthyle, Ar₂ étant en outre éventuellement substitué par un ou plusieurs radicaux alkyle linéaires ou ramifiés, de préférence en C1-C6,
   R₁, R₂ et R₃ ne représentant pas tous simultanément un atome d'hydrogène, et dans lequel chaque composé de formule générale (I) particulier est porté par, en particulier imprégné sur, des particules d'au moins deux différents types, les particules d'un même type et portant un même composé de formule générale (I) étant disposées sur un support solide distinctement des autres particules du système.

On entend par là que dans le système de détection selon l'invention, chaque composé particulier répondant à la formule générale (I) est porté par au moins deux différents types de particules. Le système comprend ainsi, pour chaque composé particulier répondant à la formule générale (I), par exemple un composé A, au moins un premier couple « composé particulier A / premier type de particules » et un deuxième couple « composé particulier A / deuxième type de particules différent du premier type de particules ». Ces deux couples sont déposés sur un support solide, distinctement l'un de l'autre, c'est-à-dire, soit sur deux zones distinctes d'un même support solide, soit sur deux supports solides distincts réunis dans le même système de détection. Plus généralement, au sein du système de détection selon l'invention, chaque composé de formule (I) particulier est associé à plusieurs types de particules différents. Comme indiqué ci-avant, les propriétés optiques de chacun des composés de formule générale (I) particuliers sont différentes selon le type de particules qui les portent. En particulier, leurs réponses colorimétrique et/ou fluorogénique provoquées par leur mise en contact avec une substance chimique sont affectées différemment selon ce type de particules. La mise en oeuvre, au sein d'un seul et même système de détection conforme à l'invention, de plusieurs types de particules différents pour un même composé de formule générale (I) particulier, permet par conséquent d'améliorer la précision de détection de substances cibles dans un milieu, par rapport aux systèmes de détection de l'art antérieur dans lesquels chaque composé est mis en oeuvre porté par un seul type de particules.

On entend dans la présente description, par hétérocycle aromatique azoté, de manière classique en elle-même, un cycle aromatique comportant au moins un atome d'azote dans le cycle, par exemple un ou deux atomes d'azote dans le cycle. Cet hétérocycle aromatique azoté comporte de préférence 6 atomes dans le cycle, ces atomes étant en outre préférentiellement uniquement des atomes de carbone ou d'azote.

On entend en outre, par groupe aromatique polycyclique condensé, un groupe comprenant une pluralité de cycles aromatiques, chacun de ces cycles étant condensé avec au moins un autre de ces cycles. Préférentiellement, chacun des cycles aromatiques d'un tel groupe comporte 6 atomes dans le cycle, ces atomes étant en outre de préférence uniquement des atomes de carbone ou d'azote. Par particules de différents types, on entend des particules présentant des chimies de surface différentes les unes des autres. Ces particules peuvent être de nature chimique différente, par exemple des particules de silice et des particules d'alumine, ou être des particules de même nature chimique, mais fonctionnalisées en surface et/ou activées différemment les unes des autres, telles que : la silice simple et la silice fonctionnalisée par des groupements amine ; ou encore l'alumine neutre, l'alumine basifiée et l'alumine acidifiée.

Le système de détection selon l'invention peut avantageusement comprendre une pluralité de composés de formule générale (I) différents, chacun desdits composés étant porté par, en particulier, des particules d'au moins un, de préférence d'au moins deux, ou même plus, différents types.

Par « les particules d'un même type et portant un même composé de formule générale (I) étant disposées sur un support solide distinctement des autres », on entend dans la présente que les particules d'un même type et portant un même composé de formule générale (I) sont disposées sur un support de sorte à être séparées dans l'espace des autres particules du système, et à ne pas interférer avec elles, si bien que les résultats, en termes de réponse optique à la mise en contact avec une substance chimique cible, obtenus pour chaque couple « particules d'un même type et portant un même composé de formule générale (I) » ne sont pas altérés par ceux obtenus par les autres couples « particules d'un même type et portant un même composé de formule générale (I) ».

Ainsi, dans des modes de réalisation particuliers de l'invention, les particules d'un même type et portant un même composé de formule générale (I) particulier sont disposées, au sein du système de détection, sur un support distinct, individuel, qui leur est propre. Les différents supports peuvent alors être assemblés sur une base commune, formant ainsi un substrat de détection unique, d'utilisation plus facile.

Dans des modes de réalisation alternatifs de l'invention, l'ensemble des particules sont disposées sur un même support, les particules d'un même type et portant un même composé de formule générale (I) particulier étant disposées sur une zone distincte dudit support, qui leur est propre, sans recoupement avec la ou les zones distinctes des autres couples « particules d'un même type et portant un même composé de formule générale (I) ».

Dans de tels modes de réalisation, lesdites zones distinctes se présentent sous des formes pouvant être sensiblement circulaires, rectangulaires ou carrées.

Par « sans recoupement », il s'entend, classiquement, que la ou les zones distinctes de chacun des couples « particules d'un même type et portant un même composé de formule générale (I) » ne se mélangent pas avec la ou les zones distinctes des autres couples « particules d'un même type et portant un même composé de formule générale (I) ».

Bien entendu, tout mode de réalisation hybride entre ces deux modes de réalisation alternatifs entre également dans le cadre de l'invention, certains couples « particules / composé » du système de détection pouvant être disposés sur un même support, dans des zones distinctes de ce dernier, et d'autres sur des supports distincts.

Chacun des composés selon l'invention, répondant à la formule générale (I), est avantageusement capable de se transformer au contact d'au moins une substance chimique que l'on souhaite détecter, cette transformation étant le signe de la présence de cette substance chimique, et se matérialisant par un changement de couleur, décelable par détection colorimétrique, et/ou un changement du signal de fluorescence, décelable par détection fluorogénique, et cette transformation étant différente selon le type de particules par lesquelles le composé est porté.

Plus spécifiquement, pour chaque type de particules par lesquelles il est porté, le composé selon l'invention présente un état coloré et un signal de fluorescence initiaux donnés. En présence d'une substance chimique cible donnée, il peut présenter un autre état coloré et/ou un signal de fluorescence d'intensité différente et/ou à une longueur d'onde différente, ces changements de propriétés optiques étant variables en fonction à la fois de la substance chimique particulière en présence, et du type de particules portant le composé.

Comme indiqué ci-avant, le système selon l'invention permet ainsi, par la quantité, et le cas échéant la redondance, d'informations qu'il procure, de détecter de manière fiable et précise, et le cas échéant de discriminer, une ou plusieurs substances chimiques, en particulier sous forme liquide, le degré de fiabilité de détection obtenu étant d'autant plus important que le nombre de couples « particules d'un même type / même composé de formule générale (I) » mis en oeuvre est important. En effet, il a été observé par les présents inventeurs que chaque substance chimique à détecter est associée à un ensemble de données s'exprimant en termes de la combinaison d'une réponse / absence de réponse colorimétrique et d'une réponse / absence de réponse fluorogénique associée à chaque couple « particules d'un même type / même composé de formule générale (I) » envisageable, cet ensemble de données étant différent d'une substance chimique à une autre. Ainsi, par un choix adéquat du nombre et de la nature de tels couples mis en oeuvre, il est possible d'associer à chaque substance chimique à détecter une signature, sous forme d'un ensemble de données optiques, qui lui est propre, et qui permet, non seulement de la détecter avec un haut niveau de fiabilité, mais également de la distinguer spécifiquement des autres substances chimiques.

Le système de détection de l'invention peut notamment permettre, par le choix de couples « particules d'un même type / même composé de formule générale (I) » appropriés, la discrimination de composés chimiques distincts appartenant à la même famille, tels que, à titre d'exemple non limitatif, les composés organophosphorés (comme ceux de la série G, de la série V ou des agents neurotoxiques organophosphorés récemment ajoutés au Tableau 1 de l'annexe de la convention de l'OIAC pour l'interdiction des armes chimiques S/1821/2019/Rev.I/Add.I (20 mai 2020)), les vésicants (tels que l'ypérite soufrée, l'ypérite azotée, la léwisite), les toxiques industriels chimiques (tels que l'ammoniac, la chloropicrine), les pesticides. Dans de tels contextes, la combinaison des deux modes de détection, colorimétrique et fluorogénique, appliquée à une pluralité de couples « particules d'un même type / même composé de formule générale (I) » distincts, permet avantageusement de recueillir l'ensemble des informations essentielles à l'identification de la menace, ainsi qu'à parer à d'éventuelles interférences entre différentes substances toxiques présentes.

Le système de détection selon l'invention est notamment particulièrement adapté pour la détection en phase solide de substances chimiques présentes dans un milieu sous forme liquide.

Le système de détection selon l'invention peut en outre répondre à l'une ou plusieurs des caractéristiques décrites ci-après, mises en oeuvre isolément ou en chacune de leurs combinaisons techniquement opérantes.

La formule générale (II) ci-dessus, représentant le groupe R₄, englobe aussi bien les formes isomères trans que les formes isomères cis de la double liaison vinylique.

Préférentiellement, le composé de formule générale (I) présente une conformation trans au niveau de chacune des liaisons vinyliques formées entre Ar₁ et chacun des groupements Ar₂.

Lorsqu'Ar₁ représente un hétérocycle aromatique azoté, chacun des groupes R₄ de la molécule est de préférence branché sur Ar₁ en position ortho ou en position para par rapport à un atome d'azote de cet hétérocycle aromatique azoté. Lorsqu'Ar₁ représente un groupe aromatique polycyclique condensé, au moins un des groupes R₄ de la molécule est de préférence branché sur Ar₁ en position ortho ou en position para par rapport à un atome d'azote de l'hétérocycle aromatique azoté de ce groupe aromatique polycyclique condensé.

Dans la formule générale (I), Ar₁ est de préférence choisi parmi les groupes de formules générales (IIIa), (IIIb), (IIIc) et (IIId) : 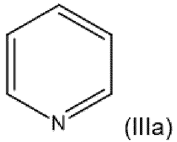 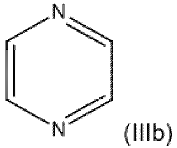 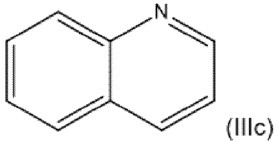 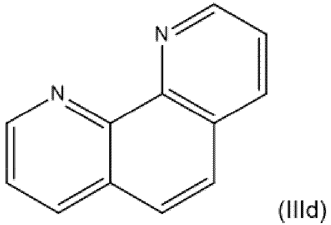 chacun de ces groupes de formules générales (IIIa), (IIIb), (IIIc) et (IIId) étant éventuellement substitué par un ou plusieurs radicaux alkyle linéaires ou ramifiés, de préférence en C1-C6, et préférentiellement en C1-C3.

Préférentiellement, dans la formule générale (II), Ar₂ représente un groupe phényl substitué par ledit groupement Rs en position para par rapport à la liaison vinylique à Ar₁. La substitution par le groupement Rs peut autrement être réalisée en position ortho, ou en position méta, par rapport à la liaison vinylique à Ar₁. Pour chacun des groupes R₁, R₂ et R₃ ne représentant pas un atome d'hydrogène, Rs représente de préférence un radical alkyle linéaire ou ramifié, en C1-C20, de préférence en C1-C6, et préférentiellement en C1-C3. Rs représente de préférence un radical méthyle.

Des composés de formule générale (I) préférés selon l'invention sont tels que :
- R₂ et R₃ représentent un atome d'hydrogène,
- ou R₁ et R₂ sont identiques et Rs représente un atome d'hydrogène,
- ou R₁, R₂ et R₃ sont identiques.

De tels modes de réalisation facilitent avantageusement la synthèse du composé de formule générale (I) entrant dans la constitution du système de détection selon l'invention.

Sont particulièrement préférés dans le cadre de l'invention les composés de formules générales respectives (IV), (V) et (VI) : 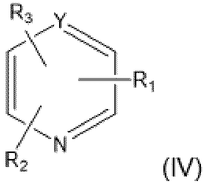 dans laquelle Y représente un atome d'azote ou, préférentiellement, un groupe CH, 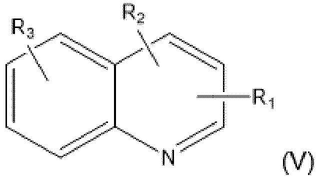 ou 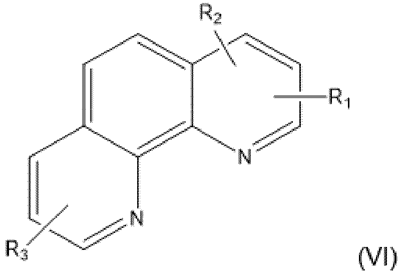 les substituants R₁, R₂ et R₃ étant tels que défini ci-avant.

Plus particulièrement, des composés de formule générale (I) peuvent répondre aux formules générales respectives (IVa), (IVb), (Va), (Vb), (Vc), (VIa), (Vlb), (VIc), dans lesquels Rs est tel que défini ci-avant : 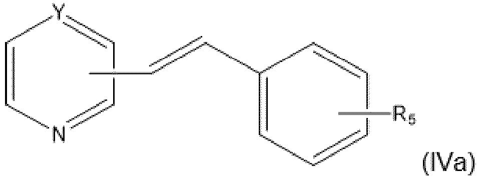
dans laquelle Y représente un atome d'azote ou un groupe CH, et Rs est de préférence branché sur le groupe phényle en position para par rapport à la liaison vinylique, 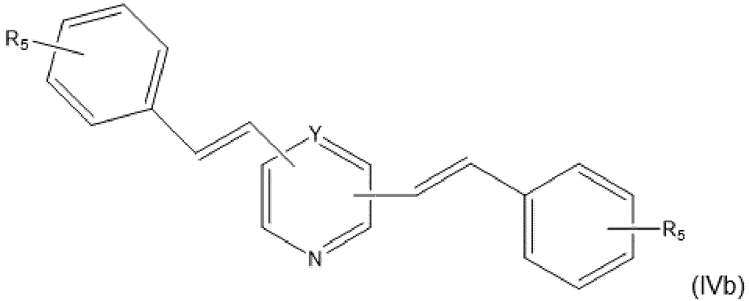
dans laquelle Y représente un atome d'azote ou, préférentiellement, un groupe CH, et chacun des groupes Rs est de préférence branché sur le groupe phényle en position para par rapport à la liaison vinylique, 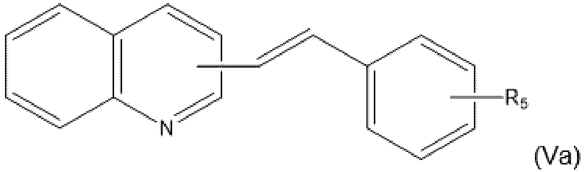
dans laquelle Rs est de préférence branché sur le groupe phényle en position para par rapport à la liaison vinylique, 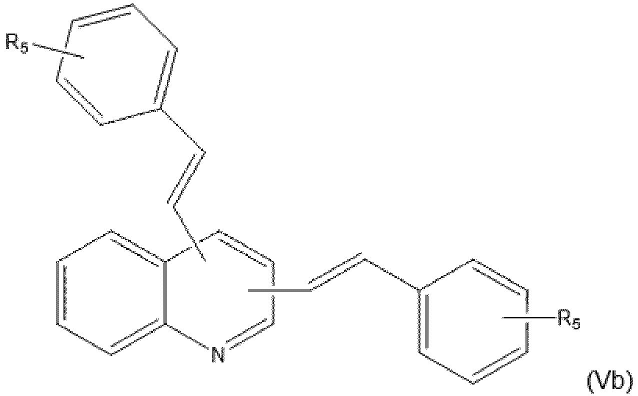
dans laquelle chacun des groupes Rs est de préférence branché sur le groupe phényle en position para par rapport à la liaison vinylique, 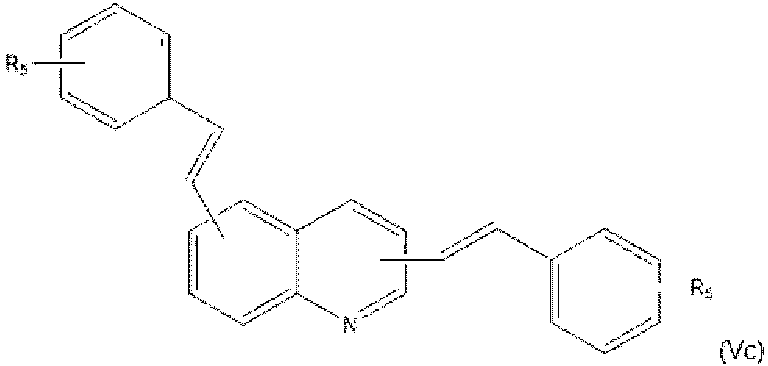
dans laquelle chacun des groupes Rs est de préférence branché sur le groupe phényle en position para par rapport à la liaison vinylique, 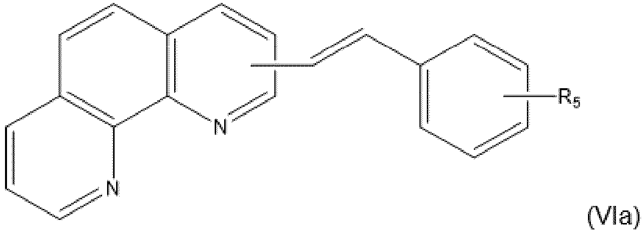
dans laquelle Rs est de préférence branché sur le groupe phényle en position para par rapport à la liaison vinylique, 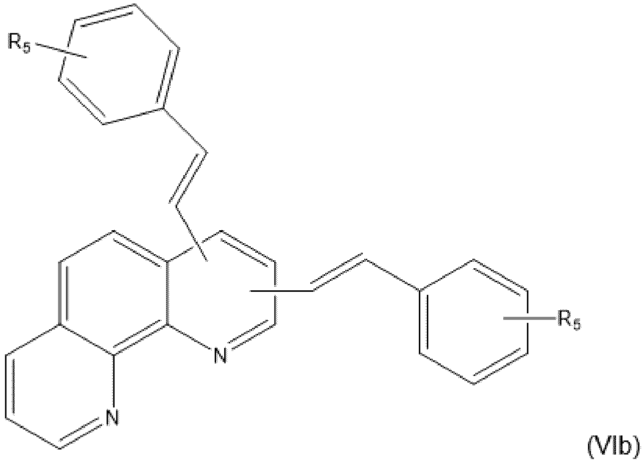
dans laquelle chacun des groupes Rs est de préférence branché sur le groupe phényle en position para par rapport à la liaison vinylique, 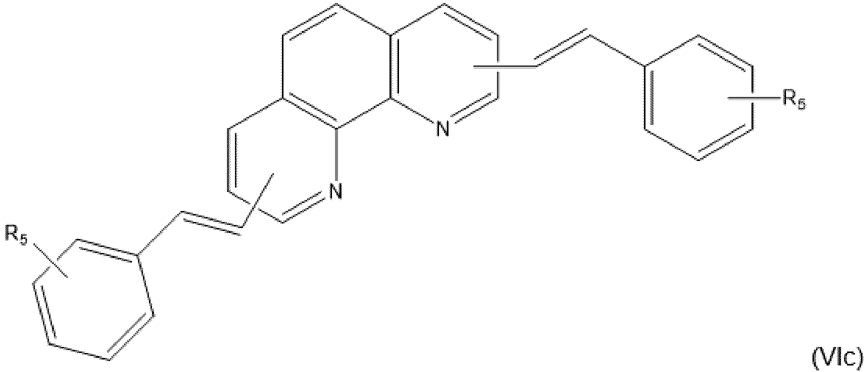
dans laquelle chacun des groupes Rs est de préférence branché sur le groupe phényle en position para par rapport à la liaison vinylique.

Dans l'ensemble des formules générales (IVa), (IVb), (Va), (Vb), (VIa), (Vlb), (VIc) ci-dessus, le groupement de formule générale (IIa) ou chacun des groupements de formule générale (IIa) : 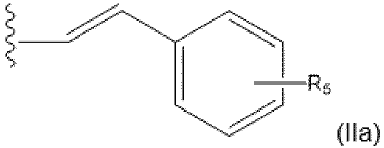 est de préférence branché en position ortho ou para d'un atome d'azote de l'hétérocycle aromatique azoté.

Des composés particuliers de formule générale (Vc) répondent en outre à la formule générale (Vc') :

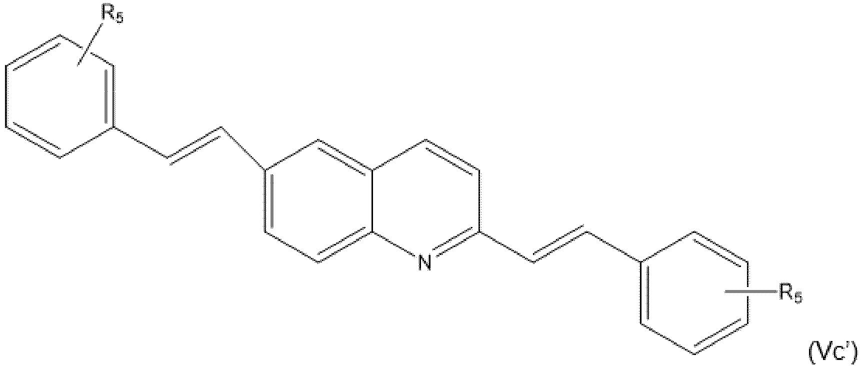

Dans l'ensemble des formules générales ci-dessus, chacun des groupes Rs représente en outre de préférence un atome d'hydrogène, un atome d'halogène et un groupement -R₆, -NHR₆, -N(R₆)₂, -OR₆ ou -SR₆, où R₆ représente un radical alkyle linéaire ou ramifié en C1-C6, de préférence en C1-C3, et préférentiellement un radical méthyle.

Les composés de formule générale (I) selon l'invention peuvent être achetés chez un fournisseur ou peuvent être synthétisés par des techniques classiques de synthèse organique impliquant, par exemple, des réactions de couplage, comme un couplage de Heck, un couplage de Suzuki, ou des réactions impliquant l'utilisation de micro-ondes.

Les composés de formule générale (I) selon l'invention peuvent notamment être obtenus par une réaction de condensation de type Knoevenagel, par exemple en adaptant, au composé particulier visé, les protocoles opératoires décrits dans les publications suivantes : Brasselet et al., Chem. Mater. 1999, 11 (7), 1915-1920 ; Cherioux et al., Chem. Mater. 1998, 10, 1984-1989 ; Bonaccorso et al., ChemPhysChem, 2018, 19 (15), 1917-1929.

Les particules mises en oeuvre dans le système de détection selon l'invention peuvent être des particules inorganiques, des particules organiques ou des particules hybrides organiques-inorganiques, par exemple, des particules dites « coeur-coquille » avec un coeur inorganique et une coquille organique.

A titre d'exemples de particules inorganiques, il peut être fait mention :
- de particules de silice SiO₂ ;
- des particules de silice greffées par des groupes organiques, par exemple, des particules de silice greffées par des groupes amine primaire -NH₂ ou des groupes aminopropyle ;
- de particules d'alumine ;
- de particules d'oxyde de zinc ;
- de particules d'oxyde de titane ;
- de particules de terre de diatomée ;
- de particules de zéolithe ;
- de particules en géopolymère.

A titre d'exemples de particules organiques, il peut être fait mention de particules polymériques, telles que :
- des particules de polyéthylène ;
- de particules de polyamide, telles que des particules de nylon ;
- de particules en biopolymère, telles que des particules d'amidon, des particules de cellulose, des particules d'agar-agar.

Par ailleurs, les particules, de manière générale, doivent présenter, avantageusement, une taille moyenne de particules compatible pour une utilisation, avant intégration dans le système de détection, dans une composition destinée à être déposée sur un support et, par exemple, peuvent présenter une taille moyenne de particules allant de 0,1 à 500 µm et, plus particulièrement, de 0,1 à 50 µm.

Dans des modes de réalisation particuliers de l'invention, les particules d'au moins un, de préférence plusieurs, desdits différents types de particules, et préférentiellement les particules de tous les types de particules mis en oeuvre, sont des particules inorganiques, de préférence choisies parmi les particules de silice, les particules de silice greffées par des groupes organiques, les particules d'alumine, les particules d'oxyde de zinc, les particules d'oxyde de titane, les particules de terre de diatomée, les particules de zéolithe et les particules en géopolymère.

A titre d'exemple, peuvent être mis en oeuvre dans le cadre de l'invention les différents types de particules suivants :
- les particules de silice SiO₂, par exemple présentant une taille moyenne de 2 à 25 µm et une taille de pores de 60 Å pour un volume de pores de 0,75 cm²/g ;
- les particules de silice fonctionnalisée par des groupements amines primaires SiO₂-NH₂, par exemple présentant une taille moyenne de particules de 40-75 µm avec une taille de pore de 110 Å ;
- les particules d'alumine neutre Al₂O₃-neutre, alumine activée, neutralisée (dans l'eau) à pH 7,0 +/- 0,5, Brockmann I, en particulier de grade standard, par exemple possédant une taille de pore de 58 Å et une taille de particules comprise entre 40 et 160 µm (205 m²/g) ;
- les particules d'alumine basique Al₂O₃-basique, alumine activée, basifiée (dans l'eau) à pH 9,5 +/- 0,5, Brockmann I, en particulier de grade standard, par exemple possédant une taille de pore de 58 Å et une taille de particules de 150 mesh (205 m²/g) ;
- les particules d'alumine acide Al₂O₃-acide, alumine activée, acidifiée (dans l'eau) à pH 4,5 +/- 0,5, Brockmann I, en particulier de grade standard, par exemple possédant une taille de pore de 58 Å et une taille de particules comprise entre 50 et 300 mesh (155 m²/g).

Les particules entrant dans la constitution du système de détection selon l'invention portant un composé de formule générale (I), peuvent être obtenues par imprégnation de particules par une solution dite d'imprégnation, comprenant un composé de formule générale (I) et un solvant organique, puis séchage des particules imprégnées obtenues par évaporation du solvant, notamment par distillation sous vide.

Le solvant organique mis en oeuvre est avantageusement choisi de sorte à répondre aux critères suivants :
- il doit être apte à solubiliser le composé de formule générale (I) ;
- il doit être compatible avec les particules destinées à être imprégnées par le composé, c'est-à-dire qu'il ne doit pas solubiliser ou engendrer l'agrégation des particules lors de l'imprégnation ;
- il doit être facile à évaporer après la réalisation de l'imprégnation des particules et, en particulier, il peut avantageusement présenter un point d'ébullition inférieur à 250°C à pression atmosphérique, par exemple inférieur à 100°C ;
- il doit être, de préférence, un solvant polaire pour solubiliser au mieux le composé de formule générale (I).

A titre d'exemples de solvants susceptibles d'être utilisés pour l'imprégnation des particules par un composé de formule générale (I), il peut être fait mention de solvants polaires protiques, tels que :
- les solvants alcooliques, par exemple l'éthanol, l'isopropanol, le 2-butoxyéthanol, l'alcool benzylique, l'éthylène glycol, le 1-hexanol, le 2-isopropoxyéthanol ; et
- l'eau.

Il peut être fait mention également de solvants polaires aprotiques, tels que :
- les solvants cétoniques, tels que la cyclopentanone, la γ-butyrolactone, la 2-butanone, l'acétone ;
- les solvants éthers, tels que le tétrahydrofurane, le 1,2- diméthoxyéthane ;
- et les solvants nitriles, tels que l'acétonitrile.

La concentration en composé de formule générale (I) dans la solution d'imprégnation peut par exemple être égale à 0,25 g/l.

La quantité massique de composé de formule générale (I) peut en outre par exemple être supérieure ou égale à environ 0,1 % de la quantité massique de particules soumises à l'imprégnation.

Dans des modes de réalisation particuliers de l'invention, les particules sont disposées sur le support incluses dans une couche comprenant, outre ces particules, au moins un liant organique, ce qui signifie, en d'autres termes, que ladite couche est une couche en matériau composite comprenant une matrice en liant(s) organique(s), dans laquelle sont piégées les particules.

Le liant ou les liants organiques peuvent être, en particulier, des liants polymériques, de préférence apolaires, tels que :
- des liants polymériques comprenant des unités répétitives styréniques, tels que le polystyrène fourni par Supelco sous la référence de produit 81404 ; le polystyrène présentant une masse moléculaire en masse Mw de 35000 fourni par Aldrich sous la référence de produit 331651 ; le polystyrène présentant une masse moléculaire en masse Mw de 350 000 et une masse moléculaire en nombre de 170 000 fourni par Aldrich sous la référence de produit 441147 ; le poly(styrène-co-méthacrylate de méthyle) présentant une masse moléculaire en masse allant de 100 000 à 150 000 fourni par Aldrich sous la référence de produit 462896 ; un copolymère bloc polystyrène-b-poly(éthylène-ran-butylène)-b-polystyrène (ran signifiant que le bloc concerné est un polymère aléatoire résultant de la polymérisation de l'éthylène et du butylène) présentant une masse moléculaire en masse de 89000 fourni par Aldrich sous la référence de produit 200565 ;
- des liants de type polydiméthylsiloxane,
- et des liants de type paraffines.

Ladite couche immobilise avantageusement les particules portant le composé de formule générale (I) sur le support, tout en les protégeant des substances aqueuses.

Chacun des supports sur lesquels sont disposées des particules portant un composé de formule générale (I) est de préférence de type poreux. Il peut s'agir d'un support formé en papier (tel que du papier pour chromatographie), d'un support en fibres intissées (par exemple, un mélange de fibres de cellulose et de fibres de polyester), étant entendu que le support doit être apte à être imprégné par les particules portant le composé, et qu'il doit de préférence être de type non fluorescent.

Chacun des supports est de préférence plan, et se présente par exemple sous forme d'une bandelette.

Un objet supplémentaire de l'invention est un substrat de détection comprenant, assemblés sur une même base, de préférence plane, une pluralité de supports tels que définis ci-avant, sur chacun desquels est disposé un couple « particules d'un même type / même composé de formule générale (I) » différent.

Un autre aspect de l'invention concerne un procédé de préparation d'un système de détection selon l'invention. Ce procédé comprend des étapes de :
- préparation d'une pluralité de compositions, dites compositions de dépôt, différentes contenant chacune, respectivement, un composé de formule générale (I) particulier porté par des particules d'un différent type de particules, au moins un solvant organique, et éventuellement au moins un liant organique,
- et dépôt de chacune de ces compositions sur un support solide, distinctement les unes des autres, c'est-à-dire distinctement dans l'espace.

L'étape de préparation de ladite pluralité de compositions de dépôt comprend, en étape préliminaire, une étape de préparation des particules portant un composé de formule générale (I), de préférence par imprégnation desdites particules par une solution d'imprégnation contenant ledit composé, comme décrit ci-avant dans la présente description. Selon le composé mis en oeuvre, elle peut comprendre en étape encore préalable la synthèse de ce composé, en particulier selon l'une des méthodes mentionnées ci-avant.

La préparation des compositions de dépôt, comprenant chacune un couple « particules d'un même type / même composé de formule générale (I) » différent, peut être réalisée par dispersion des particules portant le composé dans le ou les solvant(s) organique(s), additionné(s) le cas échéant d'un ou plusieurs liants polymériques.

Le ou les solvants organiques entrant dans la constitution des compositions de dépôt sont préférentiellement des solvants apolaires, ne permettant pas de solubiliser le composé présent dans ou à la surface des particules et ne provoquant ainsi pas la désorption des composés des particules.

Ils présentent de préférence, avantageusement, un point d'ébullition supérieur à 60°C afin d'éviter une évaporation trop rapide lors de dépôts et inférieur à 250°C afin de pouvoir sécher après dépôt des particules sur le support.

En particulier, le ou les solvants organiques peuvent être des solvants hydrocarbures et, plus spécifiquement :
- des solvants aliphatiques saturés, tels que l'hexane, cyclohexane, l'heptane, l'octane, le nonane, le décane, l'undécane, le dodécane, l'isane et leurs isomères ;
- des solvants aromatiques, tels que le naphtalène, le toluène, le xylène ;
- des solvants terpénoïdes, tels que le pinène, le carène, le limonène.

Le ou les liants organiques sont de préférence des liants polymériques, notamment tels que décrits ci-avant en référence à la couche immobilisant les particules sur le support.

En particulier, le ou les liants polymériques éventuels peuvent être avantageusement des liants apolaires, tels que des liants polymériques comprenant des unités répétitives styréniques ou de type polydiméthylsiloxane. Les compositions de dépôt obtenues doivent contenir suffisamment de composé de formule générale (I), et donc suffisamment de particules portant ce composé, pour obtenir des signaux optiques d'intensité maximale, notamment une densité de coloration maximale. Il entre dans les compétences de l'homme du métier de déterminer la concentration en particules dans la composition qui est adéquate à cet effet.

A cet égard, la formulation de chaque composition de dépôt est de préférence réalisée de façon telle que chacun des rendus colorés finaux obtenus après un virage colorimétrique déclenché par la mise en contact du composé de formule générale (I) avec des substances cibles soit significatif, c'est-à-dire que la différence de couleur (ΔE_{CIE 1994}) entre la résultante colorée initiale Rj et chacune des résultantes colorées finales Rf₁, Rf₂, etc., soit au moins supérieure à 10% de la différence de couleur maximale (ΔE_{CIE 1994max} = 140), soit ΔE_{CIE 1994max} > 14. De préférence, il est visé une différence de couleur entre Ri et les Rf₁, Rf₂, etc., supérieure à 20% de la différence de couleur maximale (ΔE_{CIE 1994max} = 140), soit ΔE_{CIE 1994max} > 28.

Il est possible d'ajuster la résultante colorée initiale Ri et chacune des résultantes colorées finales Rf₁, Rf₂, etc., en jouant sur plusieurs facteurs :
- le choix du ou des couples « particules d'un même type / même composé de formule générale (I) »,
- la quantité de composé de formule générale (I) par masse de particules ;
- le ratio massique des particules dans la composition de dépôt,
la détermination combinée de ces facteurs étant du ressort de l'homme du métier.

L'étape de dépôt des compositions de dépôt sur support solide peut être réalisée par toute technique classique en elle-même pour l'homme du métier permettant le dépôt d'une composition liquide ou pâteuse sur un support solide, telle que la sérigraphie (par exemple, à travers un masque métal de type stencil à large ouverture) ou le dépôt par enduction à la racle (connu également sous l'appellation anglo-saxonne « doctor blade coating »).

Les compositions de dépôt doivent présenter une viscosité adéquate pour permettre leur dépôt sur un support solide par de telles techniques, cette viscosité pouvant être facilement réglée par l'homme du métier, par détermination de la quantité adéquate de particules, et le cas échéant la quantité adéquate de liant organique, qui y sont contenues.

L'étape de dépôt des compositions de dépôt sur support solide est de préférence suivie d'un séchage, en particulier à l'air libre, de sorte à assurer l'évaporation du ou des solvants organiques.

Chaque support mis en oeuvre dans le procédé de préparation selon l'invention peut répondre à l'une ou plusieurs des caractéristiques décrites ci-avant en référence au système de détection selon l'invention. Il est de préférence plan, et par exemple formé en papier.

Dans des modes de mise en oeuvre particuliers de l'invention, le dépôt des différentes compositions de dépôt sur support solide est réalisé respectivement sur des zones distinctes d'un même support, chacune des compositions étant déposée sur une zone du support ne se recoupant pas avec les zones de dépôt des autres compositions.

Dans des modes de mise en oeuvre alternatifs de l'invention, le dépôt des compositions de dépôt sur support solide est réalisé respectivement sur des supports individuels distincts. Le procédé selon l'invention comprend alors de préférence une étape finale d'assemblage des différents supports distincts, chacun supportant un couple « particules d'un même type / même composé de formule générale (I) » spécifique, sur une base commune, de préférence plane. Là encore, toute formule hybride entre ces deux modes de mise en oeuvre entre dans le cadre de l'invention.

Le système de détection obtenu à l'issue du procédé de préparation selon l'invention regroupe ainsi au moins deux (et le cas échéant tout nombre supérieur à deux) différents types de particules associés à un même composé de formule générale (I). Dans les modes de réalisation particuliers dans lesquels il comporte un ou plusieurs composés de formule générale (I) additionnels, chacun de ces composés additionnels peut être porté par des particules d'un même type, ou, respectivement, par différents types de particules, ces types de particules pouvant être identiques, ou différents, par rapport aux types de particules portant le 1^{er} composé de formule générale (I).

Le système de détection selon l'invention peut ainsi contenir une pluralité de couples « particules d'un même type / même composé de formule générale (I) » tous différents les uns des autres, chacun immobilisé sur un support séparément des autres dans l'espace.

Les propriétés optiques, notamment colorimétriques et fluorogéniques, initiales de chacun de ces couples, peuvent avantageusement être déterminées, par des méthodes classiques en elles-mêmes. Leurs propriétés optiques en présence de substances chimiques cibles prédéfinies peuvent également être prédéterminées. L'ensemble de ces données, s'exprimant en termes de variation colorimétrique ou absence de variation colorimétrique, et variation du signal de fluorescence ou absence de variation de fluorescence, peuvent être stockées dans une base de données, permettant d'associer chacune de ces substances chimiques cibles prédéfinies à une combinaison de données de réponses colorimétriques et fluorogéniques qui lui est propre, vis-à-vis d'un ensemble donné de couples « particules d'un même type / même composé de formule générale (I) » différents, cette combinaison de données constituant une signature spécifique à cette substance chimique cible prédéfinie, permettant de la discriminer de manière fiable par rapport à d'autres substances chimiques cibles également prédéfinies.

A cet égard, un aspect supplémentaire de l'invention concerne un kit de détection, et le cas échéant de discrimination, ou pré-identification, d'une ou plusieurs substances chimiques, en particulier sous forme liquide, susceptible d'être contenue dans un milieu, ce kit comportant :
- un système de détection selon l'invention,
- et une base de données colorimétriques et fluorogéniques permettant de faire la correspondance entre les réponses colorimétriques et les réponses fluorogéniques observées pour chaque couple « particules d'un même type / même composé de formule générale (I) » différent compris dans ledit système de détection, et chacune desdites substances chimiques.

Le système de détection selon l'invention, et le kit qui le contient, sont particulièrement adaptés pour la détection, et même la pré-identification, d'une ou plusieurs substances chimiques présentes sous forme liquide dans un milieu. Ainsi, un autre aspect de l'invention concerne un procédé de détection d'une substance chimique, en particulier liquide, susceptible d'être contenue dans un milieu, comprenant des étapes de :
- mise en contact dudit milieu avec l'ensemble des particules portant un composé de formule générale (I) d'un système de détection selon l'invention ;
- acquisition de la réponse colorimétrique et de la réponse fluorogénique induites par ladite mise en contact, pour chaque couple « particules d'un même type / même composé de formule générale (I) » différent compris dans ledit système de détection,
- déduction, en fonction de l'ensemble desdites réponses colorimétriques et desdites réponses fluorogéniques, de l'absence ou de la présence, le cas échéant spécifique, de ladite substance dans ledit milieu.

Le procédé selon l'invention peut comprendre une étape préalable de sélection adéquate des différents couples « particules d'un même type / même composé de formule générale (I) » entrant dans la constitution du système de détection, en fonction de la substance chimique particulière dont la présence est suspectée dans le milieu, et qui est à détecter. Cette sélection est de préférence réalisée de sorte à permettre de discriminer, par le procédé selon l'invention, cette substance chimique vis-à-vis d'autres substances chimiques de même type, dont la présence est également suspectée. Ainsi, préférentiellement, l'ensemble des couples « particules d'un même type / même composé de formule générale (I) » entrant dans la constitution du système de détection sont choisis pour que la combinaison de leurs réponses colorimétriques et fluorogéniques en présence de la substance chimique cible soit spécifique de cette dernière. Comme indiqué ci-avant, un nombre d'autant plus élevé de couples « particules d'un même type / même composé de formule générale (I) » différents, choisis de manière adéquate, permet d'augmenter la fiabilité de discrimination de la substance chimique cible.

L'étape de mise en contact du milieu avec les particules du système de détection est réalisée de sorte à ce que chaque couple « particules d'un même type / même composé de formule générale (I) » soit mis en contact avec le milieu. Cette mise en contact peut être réalisée par dépôt, en surface de chaque support ou de chaque zone du support supportant des particules portant un composé de formule générale (I), sur ces dernières, d'une ou plusieurs gouttes du milieu à analyser. Elle peut autrement par exemple être réalisée par frottement du ou des supports contre une surface suspectée d'être contaminée par la substance chimique cible.

Entre l'étape de mise en contact et l'étape d'acquisition des réponses optiques induites par la mise en contact des particules portant les composés avec le milieu analysé, il peut être prévu un temps d'attente pour que, le cas échéant, le milieu ait le temps de pénétrer jusqu'aux composés de formule générale (I) portés par les particules immobilisées dans la couche de liant organique, le virage chromatique et/ou le décalage du signal de fluorescence se produisant alors quant à eux généralement quasi-instantanément.

L'étape d'acquisition de la réponse colorimétrique et de la réponse fluorogénique induites par la mise en contact avec le milieu à analyser de chaque couple « particules d'un même type / même composé de formule générale (I) » différent peut avantageusement être réalisée de façon simple et rapide, qui plus est au moyen d'appareillage usuel peu lourd et encombrant, facile à transporter.

Les réponses colorimétriques peuvent notamment être observées de manière visuelle, à l'œil nu. Elles peuvent autrement être déterminées par lecture instrumentée des spectres UV-visibles de chaque couple « particules d'un même type / même composé de formule générale (I) ».

Les réponses fluorogéniques peuvent également être observées de manière visuelle, à l'œil nu, sous éclairage par une lampe UV, à une longueur d'onde adéquate, par exemple à 312 nm ou 365 nm. Elles peuvent autrement être évaluées au moyen d'un spectromètre, notamment portable, sous longueur d'onde d'excitation adéquate, par exemple à 312 nm ou 365 nm, un tel moyen d'acquisition présentant l'avantage de procurer des résultats quantifiables, augmentant par là-même la précision de détection et pré-identification de la substance chimique en présence.

Les données colorimétriques et spectrales ainsi obtenues caractérisent, pour chaque couple « particules d'un même type / même composé de formule générale (I) » du système de détection, le virage de couleur ou l'absence de virage (qui constitue également une information), ainsi que le changement d'émission de fluorescence ou l'absence de tel changement (qui constitue également une information). La combinaison de ces données constitue la signature optique de la substance chimique cible, associée à sa structure chimique particulière.

La déduction de la présence ou de l'absence spécifique de la substance cible dans le milieu peut alors être réalisée par comparaison de l'ensemble des réponses colorimétriques et fluorogéniques obtenues avec une base de données préétablie. Cette comparaison peut être réalisée de manière manuelle, ou par des moyens informatiques appropriés.

Comme indiqué ci-avant, par un choix adéquat du nombre et du type de couples « particules d'un même type / même composé de formule générale (I) » différents mis en oeuvre, il est possible de de déceler la présence d'une substance chimique cible, et de pré-identifier la famille chimique de cette substance, et même cette substance elle-même.

La substance chimique cible peut être :
- un composé toxique de guerre, tel qu'un toxique NRBC-E (nucléaire, radiologique, biologique, chimique et explosif), un vésicant ou un agent vomitif tel qu'un composé arsénié,
- un composé toxique industriel chimique
- et/ou un pesticide.

Il peut notamment s'agir d'une substance organophosphorée.

Plus spécifiquement, des substances chimiques aptes à être détectées, et le cas échéant pré-identifiées, au moyen d'un système de détection selon l'invention, et du procédé qui le met en oeuvre, peuvent être :
- de produits phytosanitaires organophosphorés, tels que le malathion, le fenthion, le chlorpyrifos, l'ométhoate, le diméthoate, le phosalone, le dichlorvos, le trichlorfon, le phosmet, la phosalone, le parathion, l'éthéphon, le DPCP et le paraoxon-méthyl ;
- des composés toxiques de guerre organophosphorés, notamment interdits par l'Organisation pour l'interdiction des Armes Chimiques (OIAC), tels que les agents-G, les agents-V, par exemple le sarin, le tabun, le soman, le VX, le phosphonamidofluoridate de méthyl(1-(diéthylamino)éthylidène), le phosphoramidofluoridate d'éthyl(1-(diéthylamino)éthylidène), le (bis(diéthylamino)méthylène)phosphoramidofluoridate de méthyle, le VX ou les Novitchoks (A-230, A-232, A-234, A-242, etc.) ;
- des composés toxiques industriels chimiques, tels que le L1 (léwisite), le HD (ypérite soufrée), le C1 (Clark 1), le C2 (Clark 2), le HN3 (ypérite azotée) ;
- des composés sulfures, tels que l'ypérite soufrée, un composé toxique de guerre ;
- des composés azotés, tels que l'ypérite azotée, également un composé toxique de guerre ;
- des composés arséniés, tels que la léwisite, la diphénylchlorarsine, la diphénylcyanoarsine, tous également composés toxiques de guerre,

Le système de détection selon l'invention, et le procédé qui le met en oeuvre, trouvent une application particulièrement avantageuse dans les contextes suivants :
- en cas d'accident ou d'acte criminel impliquant une substance toxique NRBC-E, se répandant dans l'environnement ;
- pour l'identification d'anciens stocks, par exemple d'anciennes solutions de produits phytosanitaires,
- en post-contamination, par exemple pour la détection voire l'identification d'une menace chimique de type organophosphoré.

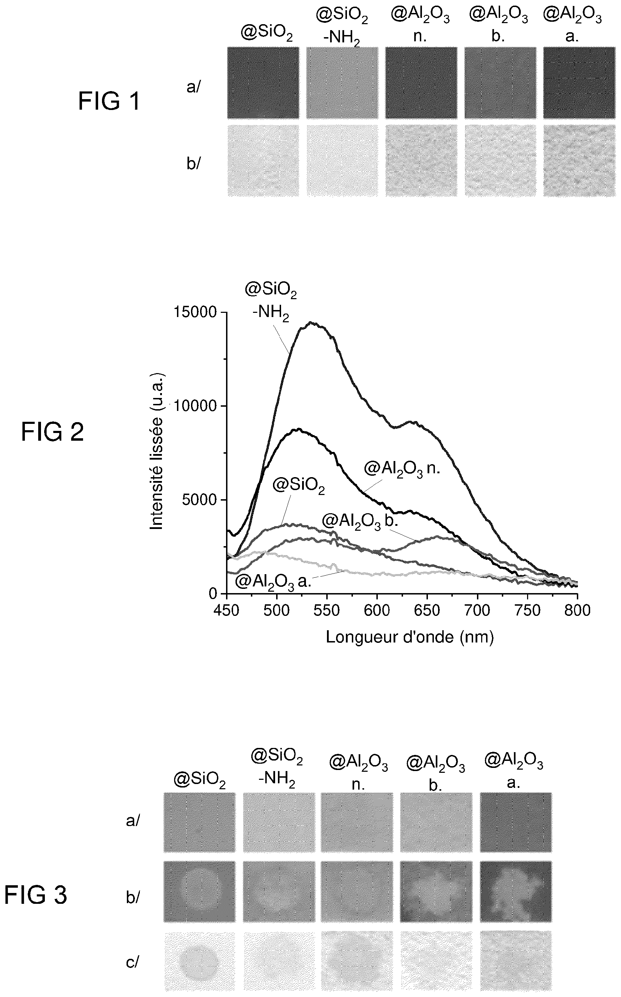

Les caractéristiques et avantages de l'invention apparaîtront plus clairement à la lumière des exemples de mise en oeuvre ci-après, fournis à simple titre illustratif et nullement limitatifs de l'invention, avec l'appui des figures 1 à 15, dans lesquelles :
La figure 1 montre des images de bandelettes conformes à l'invention portant respectivement des particules de différents types imprégnées du composé 2,4-NMe₂SQ, en a/ sous lampe UV à 312 nm, et en b/ sous la lumière visible.
La figure 2 représente les spectres de fluorescence obtenus à 310 nm pour les bandelettes de la figure 1.
La figure 3 montre des images de bandelettes conformes à l'invention portant respectivement des particules de différents types imprégnées du composé 4-NMe₂SPy, en a/, à l'état initial, sous lampe UV à 312 nm, et en b/ et c/, en présence de malathion, sous lampe UV à 312 nm (b/) et sous la lumière visible (c/).

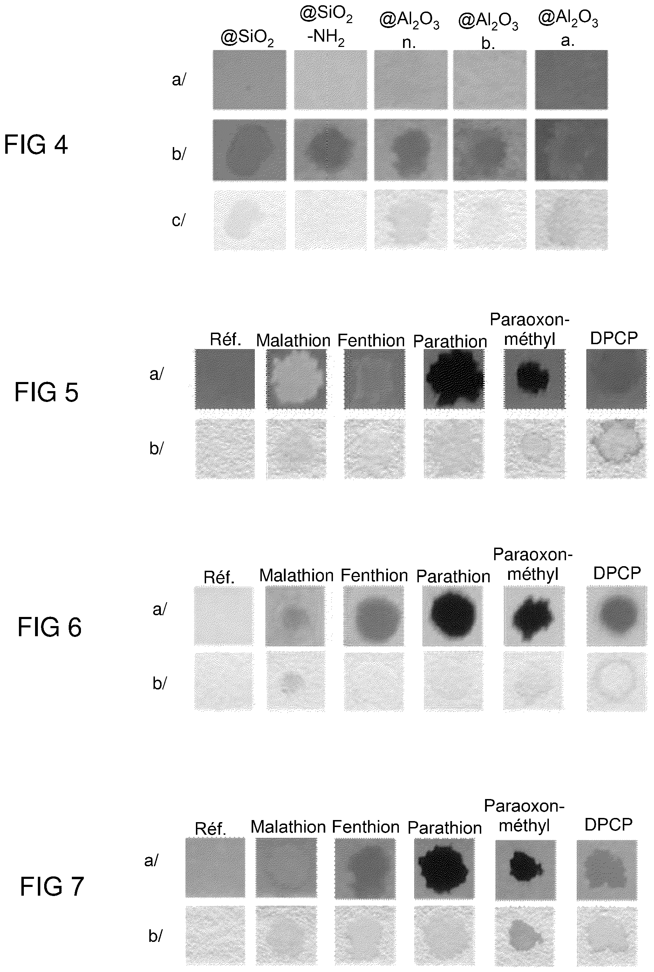

La figure 4 montre des images de bandelettes conformes à l'invention portant respectivement des particules de différents types imprégnées du composé 4-NMe₂SPy, en a/, à l'état initial, sous lampe UV à 312 nm, et en b/ et c/, en présence de fenthion, sous lampe UV à 312 nm (b/) et sous la lumière visible (c/).
La figure 5 montre des images de bandelettes conformes à l'invention portant des particules d'alumine neutre imprégnées du composé 2-NMe₂SQ, en a/ sous lampe UV à 312 nm, et en b/ sous la lumière visible, à l'état initial (Réf.) ou en présence de malathion, fenthion, parathion, paraoxon-méthyl ou DPCP.
La figure 6 montre des images de bandelettes conformes à l'invention portant des particules de silice greffée par des groupements -NH₂ imprégnées du composé 2-NMe₂SQ, en a/ sous lampe UV à 312 nm, et en b/ sous la lumière visible, à l'état initial (Réf.) ou en présence de malathion, fenthion, parathion, paraoxon-méthyl ou DPCP.
La figure 7 montre des images de bandelettes conformes à l'invention portant des particules d'alumine neutre imprégnées du composé 4-NMe₂SPy, en a/ sous lampe UV à 312 nm, et en b/ sous la lumière visible, à l'état initial (Réf.) ou en présence de malathion, fenthion, parathion, paraoxon-méthyl ou DPCP.

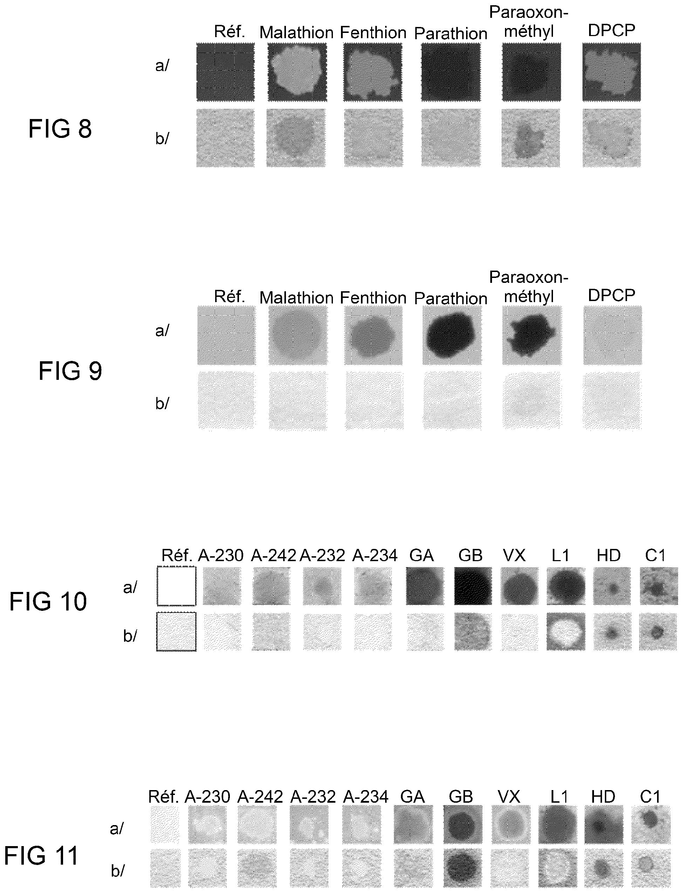

La figure 8 montre des images de bandelettes conformes à l'invention portant des particules d'alumine neutre imprégnées du composé 2,4-NMe₂SQ, en a/ sous lampe UV à 312 nm, et en b/ sous la lumière visible, à l'état initial (Réf.) ou en présence de malathion, fenthion, parathion, paraoxon-méthyl ou DPCP.
La figure 9 montre des images de bandelettes conformes à l'invention portant des particules de silice greffée par des groupements -NH₂ imprégnées du composé 2-OMeSQ, en a/ sous lampe UV à 312 nm, et en b/ sous la lumière visible, à l'état initial (Réf.) ou en présence de malathion, fenthion, parathion, paraoxon-méthyl ou DPCP.
La figure 10 montre des images de bandelettes conformes à l'invention portant des particules de silice greffée par des groupements -NH₂ imprégnées du composé 4-NMe₂SQ, en a/ sous lampe UV à 365 nm, et en b/ sous la lumière visible, à l'état initial (Réf.) ou en présence de différents Novitchoks (A-230, A-242, A-232, A-234), agents-G (GA et GB), agents-V (VX) et toxiques chimiques (L1, HD, C1).
La figure 11 montre des images de bandelettes conformes à l'invention portant des particules d'alumine basique imprégnées du composé 4-NMe₂SQ, en a/ sous lampe UV à 365 nm, et en b/ sous la lumière visible, à l'état initial (Réf.) ou en présence de différents Novitchoks (A-230, A-242, A-232, A-234), agents-G (GA et GB), agents-V (VX) et toxiques chimiques (L1, HD, C1).

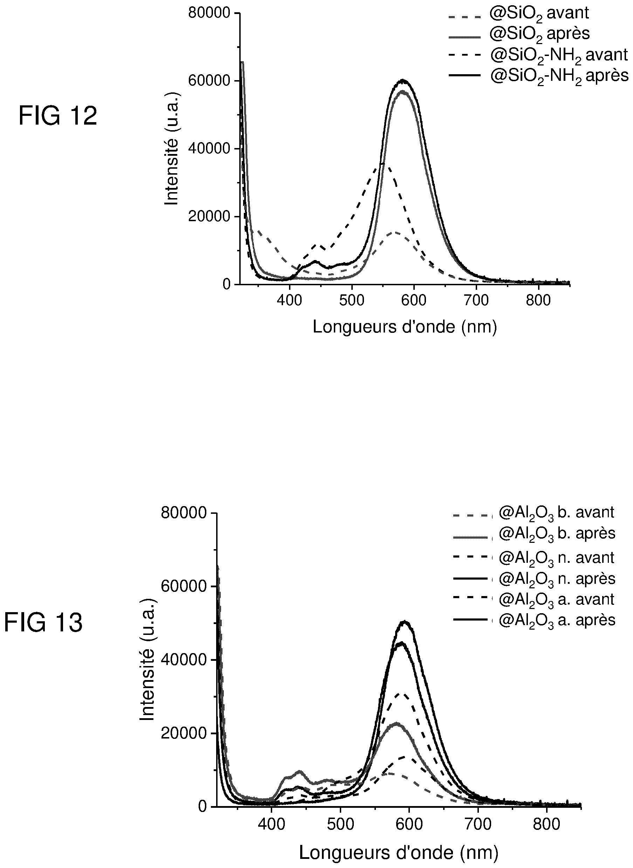

La figure 12 représente les spectres de fluorescence obtenus à 310 nm pour des bandelettes conformes à l'invention portant respectivement des particules de silice et des particules de silice greffée par des groupements -NH₂, imprégnées du composé 4-NMe₂SPy, avant ou après mise en présence de malathion.
La figure 13 représente les spectres de fluorescence obtenus à 310 nm pour des bandelettes conformes à l'invention portant respectivement des particules d'alumine neutre, des particules d'alumine basique et des particules d'alumine acide, imprégnées du composé 4-NMe₂SPy, avant ou après mise en présence de malathion.

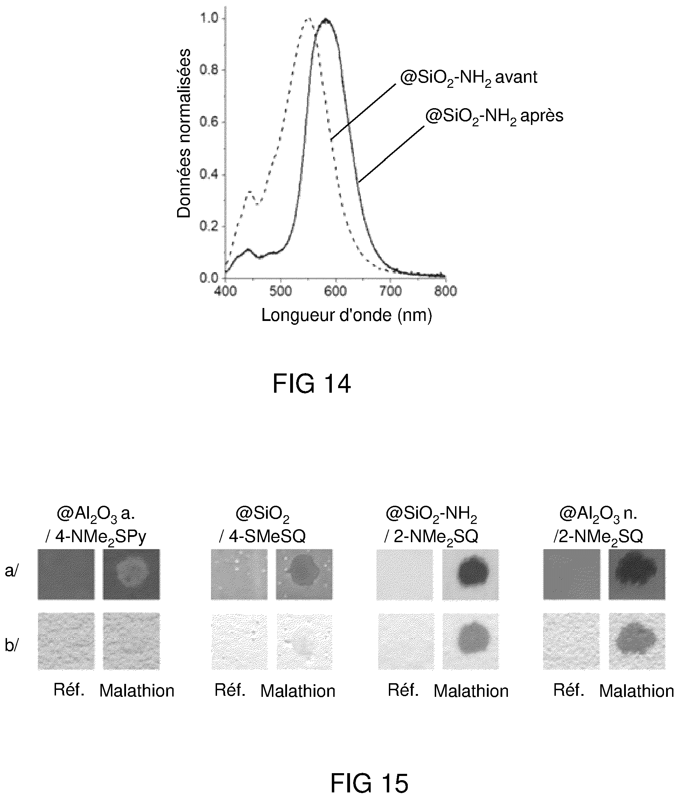

La figure 14 représente les spectres de fluorescence (données normalisées) obtenus à 310 nm pour une bandelette conforme à l'invention portant des particules de silice greffée par des groupements -NH₂, imprégnées du composé 4-NMe₂SPy, avant ou après mise en présence de malathion.
La figure 15 montre des images de différentes bandelettes conformes à l'invention portant des couples « particules / composé de formule générale (I) » différents, respectivement, « alumine acide / 4-NMe₂SPy », « silice / 4-SMeSQ », « silice greffée par des groupements -NH₂ / 2-NMe₂SQ », « alumine neutre / 2-NMe₂SQ », avant (« Réf. ») ou après (« Malathion ») exposition à une solution commerciale de malathion, en a/ sous lampe UV à 312 nm, et en b/ sous la lumière visible.

### A/ Synthèse de composés de formule générale (I)

### A.1/ Protocole général

Les composés sont préparés de la manière suivante : un volume adéquat de tert-butylate de potassium (tBuOK) (1,2 éq. pour les réactifs simplement méthylés, ou 2,4 éq. pour les réactifs di-méthylés) est prélevé en boite à gants dans un ballon de 100 mL sous atmosphère d'argon (Ar) en conditions anhydres. Le tBuOK est dissout par ajout de 5 mL de diméthylformamide (DMF) anhydre introduit à l'aide d'une seringue.

Une solution de réactifs suivants :
- un hétérocycle aromatique azoté (quinoléine, pyridine, pyrazine ou phénanthroline) méthylé ou di-méthylé (1 éq.),
- et un benzaldéhyde (1,1 éq. pour l'hétérocycle méthylé ou bien 2,2 éq. pour l'hétérocycle di-méthylé),
dans 5 mL de DMF anhydre, est introduite au goutte à goutte sur le tBuOK à température ambiante. Un changement de couleur instantané vers le marron/noir est observé.

Le milieu réactionnel est porté à 80°C toujours sous atmosphère inerte d'Ar, jusqu'à conversion complète. Un suivi en chromatographie sur couche mince (CCM) (cyclohexane:acétate d'éthyle EtOAc, 70:30) est réalisé. La bonne conversion de la réaction peut être observée dans certains cas par évolution de la coloration du mélange, généralement de couleur jaune à rouge.

La réaction est stoppée par ajout d'eau (10 à 20 mL) directement dans le milieu réactionnel. Le précipité ainsi formé est isolé par filtration sur Büchner et lavé. Les composés sont obtenus purs sans purification ou bien par recristallisation. Les spécifications propres à chacun des composés obtenus sont détaillées ci-après.

### A.2/ (E)-2-[(p-(diméthylamino)styryl]quinoléine (2-NMe₂SQ)

Ce composé, de formule : 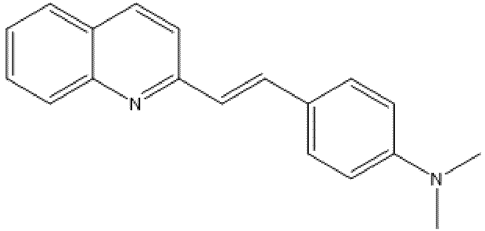 est obtenu à partir de la réaction entre la quinaldine (570 µL ; 4 mmol ; 1 éq.), le p-(diméthylamino)benzaldéhyde (0,69 g, 4,4 mmol ; 1,1 éq.) en présence de tBuOK (0,55 g ; 4,8 mmol ; 1,2 éq.) dans le DMF (10 mL). La réaction est portée à 60°C sous atmosphère inerte d'Ar jusqu'à conversion complète (1 h). La réaction est stoppée par ajout de 20 mL d'eau distillée à même le mélange réactionnel. Le précipité jaune ainsi formé est lavé à l'eau puis au cyclohexane. Le produit est obtenu pur après recristallisation dans le méthanol sous la forme d'aiguilles de couleur jaune fluo (632,5 mg ; 73,5%).
Tf : 177°C
RMN ¹H (CDCl₃, 400 MHz) : δ (ppm) 8.065 (2H, m), 7.754 (1H, d, J = 8.04 Hz), 7.679 (1H, t, J = 7.67 Hz), 7.654 (1H, d, J = 8.8 Hz), 7.62 (1H, d, J = 16.8 Hz), 7.547 (2H, d, J = 8.8 Hz), 7.455 (1H, t, J = 7.48 Hz), 7.233 (1H, d, J = 16.6 Hz), 6.735 (2H, d, J = 8.88 Hz), 3.019 (6H, s, -NMe₂)
RMN ¹³C (CDCl₃, 400 MHz) : δ (ppm) 156.887, 150.836, 136.194, 135.131, 129.669, 128.689, 127.457, 127.022, 125.672, 124.632, 124.185, 118.975, 112.221, 40.327
IR (ATR, v cm⁻¹): 3032.30 (w), 2886.33 (w), 2808.34 (w), 1599.03 (m), 1573.40 (m), 1502.91 (m), 1443.71 (m), 1355.88 (m), 1301.32 (m), 1237.51 (m), 1212.46
(m), 1185.75 (m), 1063.96 (m), 1010.61 (w), 975.62 (m), 953.88 (w), 831.84 (m), 811.17 (m), 800.54 (m), 764.88 (s), 753.78 (s)
HRMS (ASAP) m/z calculée pour [M+H]⁺ C₁₉H₁₉N₂ 275,15427 ; 275,1546 trouvée.

### A.3/ (E)-2-styrylquinoléine (2-H SQ)

Ce composé, de formule : 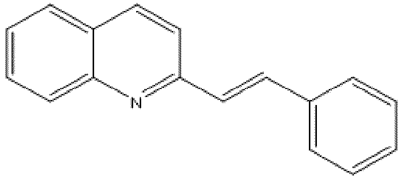 est obtenu à partir de la réaction entre la quinaldine (285 µL ; 2 mmol ; 1 éq.), le benzaldéhyde (235 µL, 2,2 mmol ; 1,1 éq.) en présence de tBuOK (275 mg; 2,4 mmol ; 1,2 éq.) dans le DMF (5 mL). La réaction est portée à 80°C sous atmosphère inerte d'Ar jusqu'à conversion complète (1 h). La réaction est stoppée par ajout de 10 mL d'eau distillée à même le mélange réactionnel. Le précipité jaune ainsi formé est lavé à l'eau. Le produit est obtenu sans purification sous la forme d'une poudre jaune fluo (334,6 mg ; 78,4%).
Tf : 98,5°C
RMN ¹H (CDCl₃, 400 MHz) : δ (ppm) 8.116 (2H, dd, J = 8.52 Hz), 7.792 (1H, d, J = 8.16 Hz), 7.677 (5H, m), 7.504 (1H, t, J = 7.20 Hz), 7.409 (3H, m), 7.331 (1H, t, J = 7.28 Hz)
RMN ¹³C (CDCl₃, 400 MHz) : δ (ppm) 156.011, 148.26, 136.539, 136.388, 134.489, 129.781, 129.205, 129.013, 128.821, 128.663, 127.521, 127.371, 127.294, 126.204, 119.275
IR (ATR, v cm⁻¹): 3056.84 (w), 3032.36 (w), 1961.62 (w), 1610.63 (m), 1593.25 (m), 1573.17 (m), 1551.27 (m), 1501.67 (m), 1444.14 (m), 1427.26 (m), 1314.36
(m), 1202.46 (m), 1117.56 (m), 1070.48 (m), 964.24 (s), 823.62 (s), 788.02 (s), 744.06 (s), 689.69 (s)
HRMS (ASAP) m/z calculée pour [M+H]⁺ C₁₇H₁₄N 232,11207 ; 232,1120 trouvée.

### A.4/ (E)-2-(p-méthylstyryl)quinoléine (2-Me SQ)

Ce composé, de formule : 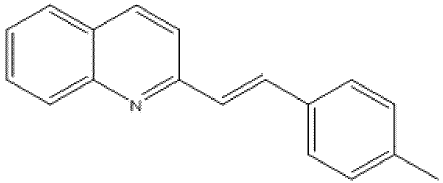 est obtenu à partir de la réaction entre la quinaldine (285 µL ; 2 mmol ; 1 éq.), le tolualdéhyde (233 µL, 2,2 mmol ; 1,1 éq.) en présence de tBuOK (275 mg ; 2,4 mmol ; 1,2 éq.) dans le DMF (5 mL). La réaction est portée à 80°C sous atmosphère inerte d'Ar jusqu'à conversion complète (2 h). La réaction est stoppée par ajout de 10 mL d'eau distillée à même le mélange réactionnel. Le précipité jaune ainsi formé est lavé à l'eau. Le produit est obtenu sans purification sous la forme d'une poudre jaune fluo (370,6 mg ; 75,5%).
Tf : 122°C
RMN ¹H (CDCl₃, 400 MHz) : δ (ppm) 8.101 (2H, dd, J = 8.49 Hz), 7.782 (H, d, J = 7.84 Hz), 7.686 (H, m), 7.546 (H, d, J = 8.03 Hz), 7.493 (H, t, J = 7.56 Hz), 7.374 (2H, d, J = 16.34 Hz), 7.216 (2H, d, J = 7.9 Hz), 2.386 (3H, s, -Me)
RMN ¹³C (CDCl₃, 400 MHz) : δ (ppm) 156.241, 148.292, 138.771, 136.301, 134 .454, 133.783, 129.719, 129.553, 129.167, 128.066, 127.500, 127.307, 127.233, 126.073, 119.217, 21.383.
IR (ATR, v cm⁻¹) : 3029.92 (w), 1695.38 (w), 1637.13 (w), 1611.50 (w), 1591.24 (m), 1550.48 (m), 1502.93 (m), 1453.76 (w), 1424.48 (m), 1303.81 (m), 1178.01 (m), 1117.53 (m), 981.54 (m), 970.61 (m), 828.55 (s), 750.33 (s)
HRMS (ASAP) m/z calculée pour [M+H]⁺ C₁₈H₁₆N 246,12772 ; 246,1278 trouvée.
A.5/ (E)-2-[p-(méthoxy)styryl]quinoléine **(2-OMe SQ)**

Ce composé, de formule : 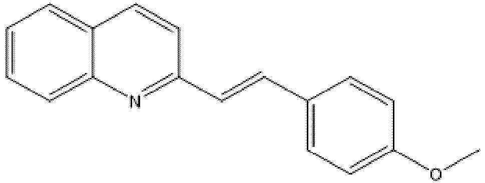 est obtenu à partir de la réaction entre la quinaldine (285 µL ; 2 mmol ; 1 éq.), l'anisaldéhyde (282 µL, 2,2 mmol ; 1,1 éq.) en présence de tBuOK (275 mg ; 2,4 mmol ; 1,2 éq.) dans le DMF (5 mL). La réaction est portée à 80°C sous atmosphère inerte d'Ar jusqu'à conversion complète (2 h et 30 min). La réaction est stoppée par ajout de 10 mL d'eau distillée à même le mélange réactionnel. Le précipité ainsi formé est lavé à l'eau. Le produit est obtenu sans purification sous la forme d'une poudre blanche (421,2 mg ; 80,6%).
Tf : 122°C
RMN ¹H (CDCl₃, 400 MHz) : δ (ppm) 8.091 (2H, dd, J = 8.54 Hz), 7.774 (H, d,
J = 8.04 Hz), 7.697 (1H, t, J = 7.4 Hz), 7.647 (1H, d, J = 16.0 Hz), 7.645 (1H, s), 7.591 (2H, d, J = 8.723 Hz), 7.483 (1H, t, J = 7.45 Hz), 7.289 (1H, d, J = 16.3 Hz), 6.940 (2H, d, J = 8.72 Hz), 3.852 (3H, s, -OMe)
RMN ¹³C (CDCl₃, 400 MHz) : δ (ppm) 160.152, 156.361, 148.269, 136.275, 134.112, 129.705, 129.328, 129.082, 128.669, 127.499, 127.234, 126.855, 125.968, 119.157, 114.287, 55.366
IR (ATR, v cm⁻¹): 2999.58 (w), 2836.14 (w), 1594.28 (s), 1551.54 (m), 1507.61 (s), 1456.50 (m), 1429.61 (m), 1301.70 (m), 1253.49 (s), 1173.37 (s), 1026.07 (s), 970.69 (s), 830.96 (s), 818.73 (s), 752.17 (s)
HRMS (ASAP) m/z calculée pour [M+H]⁺ C₁₈H₁₆NO 262,12264 ; 262,1227 trouvée.
A.6/ (E)-2-[p-(méthylthio)styryl]quinoléine **(2-SMe SQ)**

Ce composé, de formule : 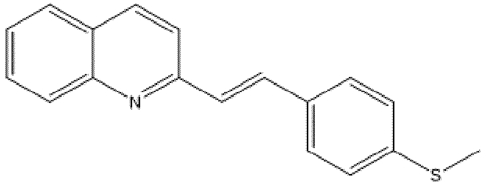 est obtenu à partir de la réaction entre la quinaldine (294 µL ; 2,2 mmol ; 1 éq.), le p-(méthylthio)benzaldéhyde (293 µL, 2,2 mmol ; 1 éq.) en présence de tBuOK (275 mg; 2,2 mmol ; 1,2 éq.) dans le DMF (5 mL). La réaction est portée à 80°C sous atmosphère inerte d'Ar jusqu'à conversion complète (4 h). La réaction est stoppée par ajout de 10 mL d'eau distillée à même le mélange réactionnel. Le précipité jaune ainsi formé est lavé à l'eau. Le produit est obtenu sans purification sous la forme d'une poudre blanche (421,3 mg ; 75,9%).
Tf : 150,5°C
RMN ¹H (CDCl₃, 400 MHz) : δ (ppm) 8.104 (2H, dd, J = 13.95 Hz & 8.56 Hz), 7,782 (1H, d, J = 8.1 Hz), 7.707 (1H, t, J = 7.9 Hz), 7.649 (2H, m), 7.562 (2H, d, J = 8.13 Hz), 7.496 (1H, t, J = 7.5 Hz), 7.356 (1H, d, J = 16.3 Hz), 7.27 (2H, d, J = 8.13 Hz), 2.52 (3H, s, -SMe)
RMN ¹³C (CDCl₃, 400 MHz) : δ (ppm) 155.943, 139.549, 136.513, 134.075, 133.264, 129.883, 128.997, 127.999, 127.521, 127.313, 126.665, 126.214, 124.099, 119.558, 119.245, 15.537
IR (ATR, v cm⁻¹): 3066.10 (w), 3023.47, 2912.29 (w), 1627.57 (w), 1609.52 (w), 1588.91 (m), 1552.85 (m), 1498.78 (m), 1402.79 (m), 1312.84 (m), 1186.09 (m), 1094.81 (m), 964.43 (s), 820.77 (s), 756.97 (s)
HRMS (ASAP) m/z calculée pour [M+H]⁺ C₁₈H₁₆NS 278,0998 ; 278,0996 trouvée.
A.7/ (E)-2-(p-bromostyryl)quinoléine **(2-BrSQ)**

Ce composé, de formule : 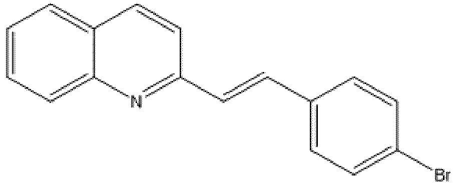 est obtenu à partir de la réaction entre la quinaldine (442 µL ; 3 mmol ; 1 éq.), le p-bromobenzaldéhyde (642,7 mg, 3,3 mmol ; 1,1 éq.) en présence de tBuOK (412,2 mg ; 3,6 mmol ; 1,2 éq.) dans le DMF (7,5 mL). La réaction est portée à 80°C sous atmosphère inerte d'Ar jusqu'à conversion complète (2 h). La réaction est stoppée par ajout de 20 mL d'eau distillée à même le mélange réactionnel. Le précipité ainsi formé est lavé à l'eau. Le produit est obtenu pur par recristallisation dans le cyclohexane sous la forme de paillettes blanches (426,7 mg ; 45,9%).
Tf : 135,5°C
RMN ¹H (CDCl₃, 400MHz) : δ (ppm) 8.17 (1H, d, J = 8.56 Hz), 8.11 (1H, d, J = 8.48 Hz), 7.82 (1H, dd, J = 8.2 & 1 Hz), 7.74 (1H, ddd, J = 8.4, 6.9 & 1.5 Hz), 7.68 (1H, d, J = 8.5 Hz), 7.67 (1H, d, J = 16.4 Hz), 7.573-7.519 (5H, m), 7.42
(1H, d, J = 16.3 Hz)
RMN ¹³C (CDCl₃, 400 MHz) : δ (ppm) 155.594, 148.288, 136.48, 135.509, 133.09, 131.982, 129.87, 129.64, 129.25, 128.69, 127.543, 127.435, 126.348, 122.552, 119.395
IR (ATR, v cm⁻¹) : 3043.88 (w), 1901.05 (w), 1612.61 (m), 1588.08 (m), 1546.86 (m), 1501.57 (m), 1484.82 (m), 1426.60 (m), 1398.49 (m), 1068.66 (m), 1004.06
(m), 967.57 (s), 819.20 (s), 743.35 (s), 490.06 (s)
HRMS (ASAP) m/z calculée pour [M+H]⁺ C₁₇H₁₃NBr 310,02259 ; 310,0225 trouvée.
A.8/ (E)-4-[p-(diméthylamino)styryl]quinoléine **(4-NMe2SQ)**

Ce composé, de formule : 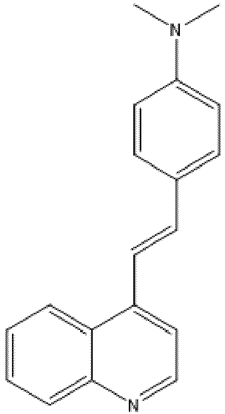 est obtenu à partir de la réaction entre la 4-méthylquinoléine (557 µL ; 4 mmol ; 1 éq.), le p-(diméthylamino)benzaldéhyde (345 mg, 4,4 mmol ; 1,1 éq.) en présence de tBuOK (550 mg ; 4,8 mmol ; 1,2 éq.) dans le DMF (10 mL). La réaction est portée à 80°C sous atmosphère inerte d'Ar jusqu'à conversion complète (6 h). La réaction est stoppée par ajout d'eau distillée à même le mélange réactionnel. Le précipité ainsi formé est lavé à l'eau/EtOH. Le produit est obtenu pur après recristallisation dans le MeOH sous la forme de cristaux jaunes (417,2 mg ; 84,3%).
Tf : 140°C
RMN ¹H (CDCl₃, 400MHz) : δ (ppm) 8.850 (1H, d, J = 4.8 Hz), 8.254 (1H, d, J = 8.0 Hz), 8.134 (1H, d, J = 8.4 Hz), 7.723 (1H, t, J = 7.04 Hz), 7.618 (1H, d, J = 16.49 Hz), 7.577 (2H, m), 7.538 (2H, d, J = 8.76 Hz), 7.331 (1H, d, J = 15.93 Hz), 6.753 (2H, d, J = 8.84 Hz), 3.038 (6H, s, -NMe₂)
RMN ¹³C (CDCl₃, 400 MHz) : δ (ppm) 150.893, 150.057, 135.416, 129.924, 129.198, 128.509, 126.45, 126.17, 124.759, 123.564, 117.629, 116.155, 112.243, 40.339
IR (ATR, v cm⁻¹): 3032.30 (w), 2886.33 (w), 2808.34 (w), 1599.03 (m), 1573.40 (m), 1502.91 (m), 1443.71 (m), 1355.88 (m), 1185.75 (m), 953.88 (m), 753.78 (s)
HRMS (ASAP) m/z calculée pour [M+H]⁺ C₁₉H₁₉N₂ 275,15427 ; 275,1545 trouvée.
A.9/ (E,E)-2,4-bis[p-(diméthylamino)styryl]quinoléine **(2,4-NMe2SQ)**

Ce composé, de formule : 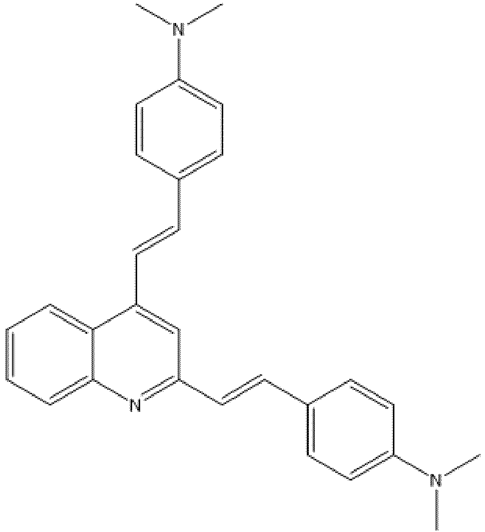 est obtenu à partir de la réaction entre la 2,4-diméthylquinoléine 46a (322 µL ; 2 mmol ; 1 éq.), le p-(diméthylamino)benzaldéhyde (690 mg; 4,4 mmol ; 2,2 éq.) en présence de tBuOK (550 mg ; 4,8 mmol ; 2,4 éq.) dans le DMF (10 mL). La réaction est réalisée à 80°C sous atmosphère inerte d'Ar jusqu'à conversion complète (3 h). La réaction est stoppée par ajout de 10 mL d'eau distillée à même le mélange réactionnel. Le précipité ainsi formé est filtré. Le produit est obtenu pur après de multiples lavages à l'EtOAc sous la forme d'un solide jaune-orange (388,9 mg ; 46,5%).
Tf : 168°C
RMN ¹H (CDCl₃, 400 MHz) : δ (ppm) 8.154 (1H, d, J = 8.3 Hz), 7.823 (1H, s), 7.704 (1H, d, J = 16.3 Hz), 7.679 (1H, t, J = 7.4 Hz), 7.589 (1H, d, J = 15.9 Hz), 7.569 (4H, t, 8.7 Hz), 7.481 (1H, t, J = 7.5 Hz), 7.367 (1H, d, J = 15.9 Hz), 7.285 (1H, d, J = 16.2 Hz), 6.749 (4H, t, J = 8.7 Hz), 3.036 (6H, s), 3.021 (6H, s) RMN ¹³C (CDCl₃, 400 MHz) : δ (ppm) 150.882, 135.344, 129.634, 128.829, 128.54, 125.46, 125.373, 124.872, 124.711, 123.44, 118.094, 114.113, 112.273, 112.228, 107.871, 40.342
IR (ATR, v cm⁻¹): 3027.49 (w), 2986.77 (w), 2894.47 (w), 2801.05 (w), 1603.33 (s), 1575.84 (s), 1523.15 (s), 1358.62 (s), 1325.92 (m), 1185.67 (s), 1168.20 (m), 958.56 (s), 805.59 (s), 764.15 (s)
HRMS (ASAP) m/z calculée pour [M+H]⁺ C₂₉H₃₀N₃ 420,24342 ; 420,2439 trouvée.
A. 10/ (E)-4-[(p-(diméthylamino)styryl]pyridine **(4-NMe₂SPy)**

Ce composé, de formule : 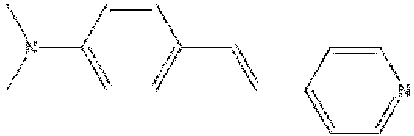 est obtenu auprès de Sigma-Aldrich (CAS : 889-36-1 ; réf. 394211-1G, lot# 04903KEV). La pureté du produit est spécifiée à 95%.

### B/ Imprégnation des particules par les composés de formule générale (I)

Le protocole opératoire général d'imprégnation des particules par les composés de formule générale (I) est le suivant :
- pesée de la poudre de particules dans un ballon ;
- pesée de la quantité de composé nécessaire (0,1% massique de composé par rapport à la masse de poudre) ;
- ajout d'un solvant (éthanol absolu) permettant la solubilisation du composé et de sorte à avoir une concentration d'environ 5 mg/20 mL;
- versement de la solution de composé dans le ballon sur la poudre et rinçage du pilulier à plusieurs reprises et versement du solvant de rinçage dans le ballon ;
- selon l'aspect de la poudre dans le ballon, rajout d'autant de solvant que nécessaire pour mouiller et mettre en suspension la poudre ;
- évaporation en trois étapes : mise en rotation dans le bain-marie (38°C) de l'évaporateur rotatif pendant 5 min pour homogénéiser la suspension dans le ballon ; descente en pression lente jusqu'à 200 mbar pour distiller lentement le solvant ; lorsque les poudres commencent à se regrouper pour former un anneau qui s'attache à la paroi du ballon, la pression est descendue plus rapidement jusqu'au séchage complet.

Les différentes particules utilisées pour les essais sont les suivantes :
- SiO₂, silice présentant une taille moyenne de particules de 2-25 µm avec une taille de pores de 60Å pour un volume de pore de 0,75 cm²/g, fournie par Aldrich sous la référence de produit 288500-1KG - cette silice est désignée « @SiO₂ » dans la présente ;
- SiO₂-NH₂, silice fonctionnalisée présentant une taille moyenne de particules de 40-75 µm avec une taille de pore de 110 Å, fournie par Aldrich sous la référence de produit 79297-100G (provenant du lot #BCCF5116) - cette silice est désignée « @SiO₂-NH₂ » dans la présente ;
- Al₂O₃-neutre, une alumine activée, neutralisée pH 7,0 +/- 0,5 (dans l'eau), Brockmann I, de grade standard, possédant une taille de pore de 58 Å, une taille de particules comprise entre 40-160 µm (205 m²/g), fournie par Aldrich sous la référence de produit 199974-1KG (provenant du lot STBB1977M9) - cette alumine est désignée « @Al₂O₃ n. » dans la présente ;
- Al₂O₃-basique, alumine activée, basifiée pH 9,5 +/- 0,5 (dans l'eau), Brockmann I, de grade standard, possédant une taille de pore de 58 Å, une taille de particules 150 mesh (205 m²/g), fournie par Aldrich sous la référence de produit 199443-1KG (provenant du lot #S32206-016) - cette alumine est désignée « @Al₂O₃ b. » dans la présente ;
- Al₂O₃-acide, alumine activée, acidifiée pH 4,5 +/- 0,5 (dans l'eau), Brockmann I, de grade standard, possédant une taille de pore de 58 Å, une taille de particules comprise entre 50-300 mesh (155 m²/g), fournie par Aldrich sous la référence de produit 199966-1KG (provenant du lot #BCD3970) - cette alumine est désignée « @Al₂O₃ a. » dans la présente.

### C/ Préparation d'une pâte d'enduction

Le protocole général de préparation d'une pâte, dite pâte d'enduction, à base de particules imprégnées d'un composé de formule générale (I), et destinée au dépôt sur un support, est le suivant.

Une solution de liant polymérique polystyrène-block-poly(éthylène-ran-butylène)-block-polystyrène (PSPEPB) est réalisée à partir de 11,5% de PSPBEPB, 61,5% de décane et 27% de cyclohexane. Les pourcentages utilisés correspondent à des pourcentages massiques. Pour réaliser 50 g de solution, on utilise ainsi 5,75 g de PSPEPB, 13,5 g de cyclohexane et 30,75 g de décane. La solution préparée est homogénéisée au bain à ultrasons pendant 1h30 à température ambiante. Elle est utilisable telle quelle après complète dissolution du PSPEPB.

Les particules imprégnées de composé sont introduites dans cette solution, à raison de 1,3 g de particules imprégnées pour 2,4 g de solution de PSPEPB, et l'ensemble est mélangé.

### D/ Enduction de la pâte d'enduction sur un support

Le protocole général d'enduction de la pâte d'enduction sur le support est le suivant.

Le support est une bandelette plane en papier pour chromatographie (Whatman 470x570 mm, cellulose grade 4 CHR, e=0,21 mm, réf.3004-917, lot # 17108889).

La réalisation de l'enduction est réalisée par raclage de la pâte d'enduction sur la surface du support par un système applicateur de film motorisé Elcometer 4340. L'étalement est réalisé de manière homogène. L'épaisseur du dépôt est réglée à 10 µm et la vitesse d'enduction ajustée à 4.

Les particules imprégnées du composé de formule générale (I) sont ainsi immobilisées sur le support, et protégées des substances aqueuses et de toute autre substance externes à laquelle le système est susceptible d'être exposé et pouvant impacter sa bonne conservation.

Pour ces exemples, chaque couple « particules d'un même type / même composé de formule générale (I) » est déposé sur bandelette individuelle qui lui est propre. Les différentes bandelettes sont assemblées sur une base commune plane.

### E/ Essais de détection / identification d'analytes

Pour ces essais, la lecture colorimétrique est réalisée par observation visuelle. La lecture de la fluorescence est réalisée par observation visuelle, sous lampe ultraviolet (UV) émettant à une longueur d'onde de 312 nm ou 365 nm, ou à l'aide d'un spectromètre portable à une longueur d'onde d'excitation de 310 nm. Les propriétés optiques, aussi bien colorimétriques que fluorimétriques, de chaque bandelette utilisée sont connues dans son état initial, avant mise en contact avec le ou les analytes à détecter.

A titre d'exemple, la figure 1 montre l'aspect visuel de bandelettes à base de différents types de particules imprégnées du composé 2,4-NMe₂SQ, en a/, observé sous la lampe UV à 312 nm, et en b/, à la lumière visible. La figure 2 montre les spectres de fluorescence obtenus au spectromètre à 310 nm. On observe que les propriétés optiques des bandelettes varient en fonction du type de particules utilisé.

Pour chacun des exemples ci-dessus, la mise en contact de l'analyte avec la bandelette s'effectue par dépôt à la micropipette de 1,2 µL d'analyte liquide pur sur la surface de la bandelette enduite des particules conformes à l'invention, imprégnées d'un composé de formule générale (I). L'observation des propriétés optiques s'effectue 1 h après ce dépôt.

### E. 1/ Essai 1 - détection du malathion

Le composé utilisé est le 4-NMe₂SPy. Les 5 types de particules ci-dessus sont testés. La lecture de la fluorescence est réalisée par observation visuelle, sous lampe ultraviolet (UV) émettant à une longueur d'onde de 312 nm.

Les résultats obtenus sont montrés sur la figure 3.

Le composé 4-NMe₂SPy imprégné sur les différents types de particules permet l'obtention de bandelettes allant de blanc à orange. Le dépôt de malathion pur initie un virage colorimétrique de couleur jaune à orange. La réponse en fluorescence est orange plus ou moins intense en fonction du type de particules utilisé. Dans cet exemple, les particules à privilégier pour la détection du malathion par 4-NMe₂SPy sont @SiO₂ et @SiO₂-NH₂, et @Al₂O₃ a..

### E.2/ Essai 2 - détection du fenthion

Le composé utilisé est le 4-NMe₂SPy. Les 5 types de particules ci-dessus sont testés. La lecture de la fluorescence est réalisée par observation visuelle, sous lampe ultraviolet (UV) émettant à une longueur d'onde de 312 nm.

Les résultats obtenus sont montrés sur la figure 4.

Le composé 4-NMe₂SPy imprégné sur les différents types de particules permet l'obtention de substrats de couleurs allant de blanc à orange. Le dépôt de fenthion pur initie un virage colorimétrique de couleur jaune pâle à orange. La réponse en fluorescence est de coloration variable à opaque plus ou moins intense en fonction du type de particules utilisé. Dans cet exemple, les particules à privilégier pour la détection du fenthion avec 4-NMe₂SPy sont @SiO₂ et @SiO₂-NH₂, et @Al₂O₃ n..

### E.3/ Essai 3 - détection d'une pluralité d'analytes organophosphorés

Le composé utilisé est le 2-NMe₂SQ. Les particules sont de l'alumine neutre @Al₂O₃ n.. La lecture de la fluorescence est réalisée par observation visuelle, sous lampe ultraviolet (UV) émettant à une longueur d'onde de 312 nm.

Les analytes suivants sont mis en présence des bandelettes : malathion, fenthion, parathion, paraoxon-méthyl, diphényl chlorophosphate (DPCP).

Les résultats obtenus sont montrés sur la figure 5.

La mise en présence des bandelettes avec les analytes purs initie un virage colorimétrie de couleur jaune ou rose en fonction de l'analyte. La réponse en fluorescence est de coloration variable elle aussi : vert pour le malathion et le fenthion, noir/opaque pour le parathion et le paraoxon-méthyl ou violet pour le DPCP.

Ces résultats montrent que ce couple composé / particules permet une détection sélective du DPCP.

On observe en outre des comportements similaires, et caractéristiques (tâche noire caractéristique observable par lampe UV) du parathion et du paraoxon-méthyl. Ce couple « particules / composé » permet ainsi de détecter de manière précise les composés organophosphorés comportant un groupement para-nitrophénol.

### E.4/ Essai 4 - détection d'une pluralité d'analytes organophosphorés

Le composé utilisé est le 2-NMe₂SQ. Les particules sont de la silice greffée @SiO₂-NH₂. La lecture de la fluorescence est réalisée par observation visuelle, sous lampe ultraviolet (UV) émettant à une longueur d'onde de 312 nm.

Les analytes suivants sont mis en présence des bandelettes : malathion, fenthion, parathion, paraoxon-méthyl, diphényl chlorophosphate (DPCP).

Les résultats obtenus sont montrés sur la figure 6.

La mise en présence des bandelettes avec les analytes purs initie un virage colorimétrique de couleur jaune ou rose en fonction de l'analyte déposé. La réponse en fluorescence est de coloration variable elle aussi : vert clair pour le malathion, vert foncé pour le fenthion, noir/opaque pour le parathion et le paraoxon-méthyl ou violet pour le DPCP. La coloration jaune pour le paraoxon-méthyl obtenue en colorimétrie permet la différenciation du parathion et paraoxon-méthyl.

Ces résultats montrent que ce couple « particules / composé » permet une détection sélective du fenthion (pas de réponse colorimétrique mais réponse fluorogénique : coloration verte sous lampe UV) et du malathion (tâche rose et réponse fluorogénique : coloration vert clair).

### E.5/ Essai 5 - détection d'une pluralité d'analytes organophosphorés

Le composé utilisé est le 4-NMe₂SPy. Les particules sont de l'alumine neutre @Al₂O₃ n. La lecture de la fluorescence est réalisée par observation visuelle, sous lampe ultraviolet (UV) émettant à une longueur d'onde de 312 nm.

Les analytes suivants sont mis en présence des bandelettes : malathion, fenthion, parathion, paraoxon-méthyl, diphényl chlorophosphate (DPCP).

Les résultats obtenus sont montrés sur la figure 7.

La mise en présence des bandelettes avec les analytes purs initie un virage colorimétrique de couleur orange plus ou moins intense en fonction de l'analyte déposé. La réponse en fluorescence est de coloration variable elle aussi : invisible pour le malathion, grisâtre pour le fenthion, noir/opaque pour le parathion et le paraoxon-méthyl ou grisâtre pour le DPCP. La coloration orange prononcé observée pour le paraoxon-méthyl en colorimétrie permet l'identification de celui-ci en comparaison à sa réponse en fluorescence identique à celle observée pour le parathion.

### E.6/ Essai 6 - détection d'une pluralité d'analytes organophosphorés

Le composé utilisé est le 2,4-NMe₂SQ. Les particules sont de l'alumine neutre @Al₂O₃ n. La lecture de la fluorescence est réalisée par observation visuelle, sous lampe ultraviolet (UV) émettant à une longueur d'onde de 312 nm.

Les analytes suivants sont mis en présence des bandelettes : malathion, fenthion, parathion, paraoxon-méthyl, diphényl chlorophosphate (DPCP).

Les résultats obtenus sont montrés sur la figure 8.

La mise en présence des bandelettes avec les analytes purs initie un virage colorimétrique très variable de couleur jaune (fenthion et parathion), rose (malathion), brun (paraoxon-méthyl) ou violet (DPCP). La réponse en fluorescence est de coloration variable elle aussi : jaune pour le malathion et le fenthion, inexistante pour le parathion et le paraoxon-méthyl ou vert/bleu pour le DPCP.

Ces résultats montrent que ce couple « particules / composé » permet une détection sélective du DPCP (réponse bleutée caractéristique sous lampe UV) ainsi que du malathion (tâche rose observable à la lumière visible couplée à une réponse fluorogénique jaune prononcée).

### E.7/ Essai 7 - détection d'une pluralité d'analytes organophosphorés

Le composé utilisé est le 2-OMeSQ. Les particules sont de la silice greffée @SiO₂-NH₂. La lecture de la fluorescence est réalisée par observation visuelle, sous lampe ultraviolet (UV) émettant à une longueur d'onde de 312 nm.

Les analytes suivants sont mis en présence des bandelettes : malathion, fenthion, parathion, paraoxon-méthyl, diphényl chlorophosphate (DPCP).

Les résultats obtenus sont montrés sur la figure 9.

La mise en présence des bandelettes avec les analytes purs initie un virage colorimétrique uniquement pour le paraoxon-méthyl (jaune) et le DPCP (orange). La réponse en fluorescence est de coloration variable : quasiment inexistante (gris bleuté) pour le malathion et le fenthion, noir/opaque pour le parathion et le paraoxon-méthyl ou orange pour le DPCP.

Ces résultats montrent que ce couple « particules / composé » permet une détection sélective du DPCP (réponse orange caractéristique sous lampe UV) ainsi que du paraoxon-méthyl (réponse caractéristique le distinguant du parathion).

### E.8/ Essai 8 - détection d'une pluralité d'analytes toxiques NRBC-E

Le composé utilisé est le 4-NMe₂SQ. Les particules sont de la silice greffée @SiO₂-NH₂. La lecture de la fluorescence est réalisée par observation visuelle, sous lampe ultraviolet (UV) émettant à une longueur d'onde de 365 nm.

Les analytes mis en présence des bandelettes sont des composés organophosphorés de type NRBC-E : agents-A (Novitchoks : A-230, A-232, A-234 et A-242), agents-G (tabun (GA) et sarin (GB)), agents-V (S-[2-(diisopropylamino)éthyl]méthylphosphonothioate d'o-éthyle (VX)) et plusieurs toxiques chimiques (2-chloroéthényldichloroarsine, ou Léwisite (L1), sulfure de dichlorodiéthyle, ou ypérite soufrée (HD), phényldichloroarsine, ou Clark 1 (C1)). Les résultats obtenus sont montrés sur la figure 10.

La mise en présence des bandelettes avec les analytes purs initie un virage en colorimétrie soit inexistant (agents-A, agents-V, et GA) soit de couleur rose (GB, L1, HD et C1). La réponse en fluorescence (lecture à 365 nm) est de coloration variable : vert/jaune (gris bleuté) pour les agents-A, vert/bleuté pour le GA, bleu foncé pour le GB, bleu clair pour le VX, violet pour les toxiques L1, HD et C1. Une décoloration caractéristique est observée dans le cas du toxique L1 ce qui permet de l'identifier facilement par rapport aux autres composés testés.

Ces résultats montrent que pour ce couple « particules / composé » :
- les Novitchoks montrent une réponse similaire sous lampe UV (365 nm) ;
- le GB est identifié sans équivoque par colorimétrie et fluorescence (365 nm) ;
- le VX est identifié sous lampe UV (365 nm) ;
- le L1 est identifié par colorimétrie et fluorescence (365 nm).

### E.9/ Essai 9 - détection d'une pluralité d'analytes toxiques NRBC-E

Le composé utilisé est le 4-NMe₂SQ. Les particules sont de l'alumine basique @Al₂O₃ b. La lecture de la fluorescence est réalisée par observation visuelle, sous lampe ultraviolet (UV) émettant à une longueur d'onde de 365 nm.

Les analytes mis en présence des bandelettes sont des composés organophosphorés de type NRBC-E : agents-A (Novitchoks : A-230, A-232, A-234 et A-242), agents-G (GA et GB), agents-V (VX) et plusieurs toxiques chimiques (L1, HD, C1).

Les résultats obtenus sont montrés sur la figure 11.

La mise en présence des bandelettes avec les analytes purs initie un virage en colorimétrie soit inexistant à jaune pâle (agents-A, agents-V, et GA) soit de couleur rouge intense (GB) ou bien rose (L1, HD et C1). La réponse en fluorescence (lecture à 365 nm) est de coloration variable : vert/jaune intense pour les agents-A, vert/bleuté pour le GA, violet/brun pour le GB, bleu clair pour le VX, violet pour les toxiques L1, HD et C1. Une décoloration caractéristique est observée dans le cas du toxique L1 ce qui permet de l'identifier facilement par rapport aux autres composés testés.

Ces résultats montrent que pour ce couple « particules / composé » :
- les Novitchoks montrent une réponse similaire sous lampe UV (365 nm) ;
- le GB est identifié sans équivoque par colorimétrie et fluorescence (365 nm) ;
- le L1 est identifié par colorimétrie et fluorescence (365 nm).

### E.10/ Essai 10 - détection du malathion par spectromètre portable

Le composé utilisé est le 4-NMe₂SPy. L'ensemble des particules mentionnées ci-dessus sont testées.

Les spectres de fluorescence obtenus au moyen du spectromètre, avant et après exposition au malathion, sont montrés sur la figure 12 pour les silices et sur la figure 13 pour les alumine, respectivement avant et après exposition au malathion.

On observe que le malathion est facilement identifiable, car il engendre après lecture de la fluorescence une augmentation de l'intensité du signal quel que soit le type de particule utilisé. Un déplacement vers de plus fortes longueurs d'onde est en outre observé suite à l'exposition au malathion.

La détection peut être affinée par calcul du déplacement vers de plus hautes (ou plus faibles) longueurs d'onde. Cette valeur Δλ, exprimée en nm, est propre à chacun des analytes testés (signature d'un analyte) et permet d'obtenir une correspondance signal/analyte détecté. Cette méthode permet l'identification précise d'un ou plusieurs analytes.

La normalisation du signal obtenu dans le présent exemple du malathion permet de mettre en évidence le phénomène de déplacement du signal après exposition au malathion.

A titre d'exemple, sont montrés sur la figure 14 les spectres de fluorescence normalisée obtenus pour les particules @SiO₂-NH₂. On y mesure un déplacement vers de plus hautes longueurs d'onde Δλ = 29 nm provoqué par l'exposition de la bandelette au malathion.

### E.11/ Essai 11 - détection du malathion par divers couples « particules d'un même type / même composé de formule générale (I) »

Une solution commerciale de malathion (huile insecticide pour cochenilles ; Bayer), anciennement commercialisée, est déposée pure sur les différents types de couples « particules d'un même type / même composé de formule générale (I) » suivants. La lecture de la fluorescence est réalisée par observation visuelle, sous lampe ultraviolet (UV) émettant à une longueur d'onde de 312 nm.

Les résultats obtenus sont montrés sur la figure 15. Plus particulièrement :
- le couple @Al₂O₃ b. / 4-NMe₂SPy engendre une réponse colorimétrique nulle et une réponse en fluorescence orange ;
- le couple @SiO₂/ 2-SMeSQ engendre une réponse colorimétrique jaune et une réponse en fluorescence verte ;
- le couple @SiO₂-NH₂/ 2-NMe₂SQ engendre une réponse colorimétrique rose et une réponse en fluorescence violet opaque ;
- le couple @Al₂O₃ n. / 2-NMe₂SQ engendre une réponse colorimétrique rose et une réponse en fluorescence violet.

### E.12/ Essai 12 - détection du malathion par divers couples « particules d'un même type / même composé de formule générale (I) »

L'obtention par le procédé selon l'invention, d'une signature unique pour un analyte donné, par la mise en oeuvre d'une pluralité de couples « particules d'un même type / même composé de formule générale (I) », est démontrée dans cet exemple pour l'analyte organophosphoré malathion, pour les 6 différents couples suivants : « @SiO₂-NH₂/ 4-NMe₂SPy », « @Al₂O₃ b. / 4-NMe₂SPy », « @Al₂O₃ n. / 2,4-NMe₂SQ », « @SiO₂/ 2-SMeSQ », « @Al₂O₃ n. / 2-NMe₂SQ », « @Al₂O₃ a. / 2-NMe₂SQ ».

Les résultats obtenus, en termes de réponse colorimétrique et réponse en fluorescence (à 312 nm), sont résumés dans le tableau 1 ci-après.

**Tableau 1 - réponses optiques de différents couples « particules d'un même type / même composé de formule générale (I) » à l'exposition au malathion - « Aug » indique une augmentation de l'intensité du pic, « red shift » indique un déplacement bathochrome, et « blue shift » indique un déplacement hypsochrome**

| Particule / composé | @SiO₂-NH₂/4-NMe₂SPy | @Al₂O₃ b./4-NMe₂SPy | @Al₂O₃ n./2,4-NMe₂S Q | @SiO₂/2 -SMeSQ | @Al₂O₃ n./2-NMe₂SQ | @Al₂O₃ a./2-NMe₂S Q |
|---|---|---|---|---|---|---|
| Réponse colorimétriqu e | orange | orange | brun | violet | rose pâle | rose |
| Réponse fluorogénique | orange | orange | jaune | jaune | vert | jaune |
| Pic de fluorescence initial (nm) | 580 | 588 | 554 | 540 | 507 | 510 |
| Modification du pic par exposition au malathion | Aug + red shift | Aug | Aug + red shift | Aug + red shift | Aug + blue shift | Aug |
| Δλ (nm) | 29 | - | 34 | 57 | -18 | - |

L'ensemble de ces données constitue la signature caractéristique du malathion vis-à-vis des couples « particules d'un même type / même composé de formule générale (I) » mis en oeuvre.

### E.13/ Essai 13 - détection du malathion par divers couples « particules d'un même type / même composé de formule générale (I) »

L'obtention par le procédé selon l'invention, d'une signature unique pour un analyte donné, par la mise en oeuvre d'une pluralité de couples « particules d'un même type / même composé de formule générale (I) », est démontrée dans cet exemple pour l'analyte organophosphoré fenthion, pour les 3 différents couples suivants : « @Al₂O₃ b. / 2,4-NMe₂SQ », « @Al₂O₃ b. / 2-NMe₂SQ », « @Al₂O₃ n. / 2-NMe₂SQ.

Les résultats obtenus, en termes de réponse colorimétrique et réponse en fluorescence (à 312 nm), sont résumés dans le tableau 2 ci-après.

**Tableau 2 - réponses optiques de différents couples « particules d'un même type / même composé de formule générale (I) » à l'exposition au fenthion - « blue shift » indique un décalage vers les plus basses longueurs d'onde**

| Particule / composé | @Al₂O₃ b. / 2,4-NMe₂SQ | @Al₂O₃ b. / 2-NMe₂SQ | @Al₂O₃ n. / 2-NMe₂SQ |
|---|---|---|---|
| Réponse colorimétrique | jaune | rose | - |
| Réponse fluorogénique | jaune | vert | vert |
| Pic de fluorescence initial (nm) | 554 | 517 | 510 |
| Modification du pic par exposition au malathion | Blue shift | Augmentati on | Blue shift |

L'ensemble de ces données constitue la signature caractéristique du fenthion vis-à-vis des couples « particules d'un même type / même composé de formule générale (I) » mis en oeuvre.

### E.14/ Essai 14 - détection du DPCP par divers couples « particules d'un même type / même composé de formule générale (I) »

L'obtention par le procédé selon l'invention, d'une signature unique pour un analyte donné, par la mise en oeuvre d'une pluralité de couples « particules d'un même type / même composé de formule générale (I) », est démontrée dans cet exemple pour l'analyte organophosphoré DPCP, pour les 6 différents couples suivants : « @SiO₂-NH₂ / 4-NMe₂SPy », « @SiO₂-NH₂ / 2,4-NMe₂SQ », « @Al₂O₃ n. / 2,4-NMe₂SQ », « @SiO₂-NH₂ / 2-SMeSQ », « @Al₂O₃ b. / 2-NMe₂SQ », « @SiO₂-NH₂/2-OMeSQ ».

Les résultats obtenus, en termes de réponse colorimétrique et réponse en fluorescence (à 312 nm), sont résumés dans le tableau 3 ci-après.

**Tableau 3 - réponses optiques de différents couples « particules d'un même type / même composé de formule générale (I) » à l'exposition au DPCP - « Décol » indique une décoloration (tache blanche), « Dim » indique une diminution de l'intensité du pic, « Aug » indique une augmentation de l'intensité du pic, « red shift » indique un décalage vers les plus hautes longueurs d'onde, et « blue shift » indique un décalage vers les plus basses longueurs d'onde**

| Particule / composé | @SiO₂-NH₂ / 4-NMe₂SPy | @SiO₂-NH₂/ 2,4-NMe₂SQ | @Al₂O₃ n. / 2,4-NMe₂SQ | @SiO₂-NH₂ / 2-SMeSQ | @Al₂O₃ b. / 2-NMe₂SQ | @SiO₂-NH₂ / 2-OMeSQ |
|---|---|---|---|---|---|---|
| Réponse colorimétrique | Décol | Décol | violet | jaune | rose | orange |
| Réponse fluorogénique | rose | Bleu/vert | Bleu/vert | jaune | rose | orange |
| Pic de fluorescence initial (nm) | 589 | 490 | 487 | - | - | 561 |
| Modification du pic par exposition au malathion | Dim + red shift | Blue shift | Blue shift | Aug + red shift | Aug + signal à 3 maxima carac. | Red shift |
| Δλ (nm) | 38 | -46 | -33 | - | - | 77 |

L'ensemble de ces données constitue la signature caractéristique du DPCP vis-à-vis des couples « particules d'un même type / même composé de formule générale (I) » mis en oeuvre.

### E. 15/ Essai 15 - détection d'une pluralité d'analytes organophosphorés

Le couple « @Al₂O₃ n. / 2-SMeSQ » est utilisé pour établir les signatures des différents analytes organophosphorés suivants : malathion, fenthion, DPCP, parathion et paraoxon-méthyl.

Les résultats obtenus, en termes de réponse colorimétrique et réponse en fluorescence (à 312 nm), sont résumés dans le tableau 4 ci-après.

**Tableau 4 - réponses optiques du couple « @Al₂O₃ n. / 2-SMeSQ » à l'exposition à différents analytes organophosphorés - « Aug » indique une augmentation de l'intensité du pic, « red shift » indique un décalage vers les plus hautes longueurs d'onde**

| Analyte | Malathion | Fenthion | DPCP | Parathion | Paraoxon-méthyl |
|---|---|---|---|---|---|
| Réponse colorimétrique | rose | rose | rose | jaune | jaune |
| Réponse fluorogénique | jaune | vert | rose | Noir/violet | Noir/violet |
| Pic de fluorescence initial (nm) | 510 | 517 | multimaxima | - | - |
| Modification du pic par exposition au malathion | Aug | Aug + red shift | Aug + signal à 3 maxima carac. | extinction | extinction |
| Δλ (nm) | - | 7 | - | - | - |

On observe que le couple « @Al₂O₃ n. / 2-SMeSQ » permet la détection et l'identification du malathion, du fenthion et du DPCP de manière précise par interprétation de la réponse en fluorescence. La méthode colorimétrique permet uniquement la mise en évidence de la présence d'un composé organophosphoré.

La réponse colorimétrique du parathion et du paraoxon-méthyl n'est pas lisible en colorimétrie. Néanmoins, l'étude de la fluorescence (extinction) permet de démontrer la présence d'un composé étranger, différent du malathion, du fenthion et du DPCP.

### E.16/ Essai 16 - détection d'une pluralité d'analytes organophosphorés par plusieurs couples « particules d'un même type / même composé de formule générale (I) »

La détection d'une série de composés organophosphorés : malathion, ométhoate, fenthion, parathion et paraoxon-méthyl est réalisée par l'utilisation de deux couples « particules d'un même type / même composé de formule générale (I) » analysés en parallèle sous éclairage de lumière blanche pour une lecture en colorimétrie et sous excitation UV à 312 nm pour une lecture en fluorescence : « @SiO₂-NH₂/ 4-NMe₂SQ » et « @SiO₂-NH₂/ 2-OMeSQ ».

Les résultats obtenus, en termes de réponse colorimétrique et réponse en fluorescence (à 312 nm), sont résumés dans le tableau 5 ci-après.

**Tableau 5 - réponses optiques des couples « @SiO₂-NH₂ / 4-NMe₂SQ » et « @SiO₂-NH₂ / 2-OMeSQ » à l'exposition à différents analytes organophosphorés - « Réf. » indique la bandelette à l'état initial**

| | @SiO₂-NH₂/ 2-OMeSQ | | | | | |
|---|---|---|---|---|---|---|
| Composé | Réf. | malathion | ométhoate | fenthion | parathion | Paraoxon-méthyl |
| Colorimétrie | Bleu | Bleu/gris | Bleu/gris | noir | noir | orange |
| Fluorescence | Jaune pâle | - | - | - | jaune | jaune |

| | @SiO₂-NH₂/ 4-NMe₂SQ | | | | | |
|---|---|---|---|---|---|---|
| Composé | Réf. | malathion | ométhoate | fenthion | parathion | Paraoxon-méthyl |
| Colorimétrie | Jaune | rose | rose | Rose pâle | jaune | brun |
| Fluorescence | Gris | rose | rose | Orange/ rose | Gris opaque | Violet/ gris opaque |

On observe que la combinaison des deux couples de détection permet l'identification précise des pesticides parathion et paraoxon-méthyl, non-identifiables par fluorogénie uniquement. La réponse en fluorescence de ces deux pesticides, qui ont pour caractéristique commune de comporter un groupement de type p-nitrophénol, se caractérise par une extinction complète de l'intensité de la fluorescence initiale des bandelettes. La lecture de fluorescence permet ainsi la détection de la présence de cette catégorie de molécules. L'association de la lecture par colorimétrie permet la différentiation de ces molécules. En effet, le couple « @SiO₂-NH₂/ 2-OMeSQ » engendre une réponse colorimétrique jaune uniquement pour le paraoxon-méthyl. Le couple « @SiO₂-NH₂ / 4-NMe₂SQ » quant à lui initie une réponse colorimétrique unique de couleur brune pour le paraoxon-méthyl, et à une réponse colorimétrique jaune en présence de parathion.

Le couple « @SiO₂-NH₂ / 2-OMeSQ » permet en outre de discriminer la présence de tout autre organophosphoré : aucune réponse colorimétrique n'est obtenue pour le malathion, l'ométhoate, le fenthion et le parathion.

### E.17/ Essai 17 - détection d'une pluralité d'analytes toxiques NRBC-E par plusieurs couples « particules d'un même type / même composé de formule générale (I) »

Dans le but d'affiner la détection des Novitchoks (A-230, A-232, A-234, A-242), vis-à-vis notamment des composés GA, GB et VX, la combinaison des couples suivants peut être utilisée : « @Al₂O₃ b. / 4-NMe₂SQ » et « @Al₂O₃ a. / 4-NMe₂SQ ».

En effet, dans le cadre de la détection des toxiques NRBC-E, les Novitchoks peuvent être confondus avec la GA sur le couple « @Al₂O₃ b. / 4-NMe₂SQ ». Le couple « @Al₂O₃ a. / 4-NMe₂SQ » permet de différencier le GA des Novitchoks, comme montré par les résultats exposés dans le tableau 6 ci-après.

**Tableau 6 - réponses optiques des couples « @Al₂O₃ b. / 4-NMe₂SQ » et « @Al₂O₃ a. / 4-NMe₂SQ » à l'exposition à différents analytes NRBC-E**

| Couple | « @Al₂O₃ b. / 4-NMe₂SQ » | | « @Al₂O₃ a. / 4-NMe₂SQ » | |
|---|---|---|---|---|
| Eclairage | Lumière blanche | UV 365 nm | Lumière blanche | UV 365 nm |
| GA | - | Bleu/vert | Orangé | Bleu/vert |
| GB | Rouge | Violet | Rose/rouge | Violet |
| VX | - | Bleu clair | - | Bleu clair |
| Novitchoks | - | Turquoise intense | jaune | Turquoise intense |

Comme on peut l'observer, vis-à-vis du couple « @Al₂O₃ a. / 4-NMe₂SQ », les Novitchoks se distinguent de GA (émission de fluorescence bleu/vert sous excitation à 365 nm et coloration orangé sous lumière blanche) par une émission de fluorescence turquoise intense sous excitation à 365 nm et une trace jaune sous lumière blanche.

### E.18/ Essai 18 - détection d'une pluralité d'analytes toxiques NRBC-E par plusieurs couples « particules d'un même type / même composé de formule générale (I) »

Dans le but de constituer un ensemble d'identification complet des analytes d'intérêt, l'impact ainsi que l'absence d'impact de ces derniers sur différentes bandelettes de détection peuvent être enregistrés dans une base de données. De plus, certaines redondances d'informations permettent de s'assurer de la fiabilité des résultats.

Dans cet essai, trois couples « particules d'un même type / même composé de formule générale (I) » ont été utilisés :
- « @SiO₂-NH₂/4-NMe₂SQ »,
- « @Al₂O₃ b./4-NMe₂SQ »,
- « @Al₂O₃ a./4-NMe₂SQ ».

Ces trois couples font intervenir le même indicateur de détection 2,4-[4-(diméthylamino)styryl]quinoléine (4-NMe₂SQ), imprégné sur trois types de particules différentes.

Les différentes bandelettes sont mise en présence des analytes suivants : GA, GB, VX, Novitchoks (A-230, A-232, A-234, A-242), HD, tris(2-chloroéthyl)amine, ou ypérite azotée (HN3), L1, C1, diphénylcyanoarsine, ou Clark 2 (C2).

Les bandelettes sont observées :
- sous lumière blanche par le biais d'un scanner de bureautique,
- sous lumière UV à 365 nm par biais d'un appareil photo.

Les données récupérées font l'objet d'une simple observation à l'œil nu. Il est bien entendu que ces données pourraient être traitées numériquement par des relevés de coordonnées colorimétriques et que les données spectrales auraient pu être collectées par un spectrophotomètre portable.

Les résultats obtenus sont résumés dans le tableau 7 ci-après.

**Tableau 7 - réponses optiques des couples « @SiO₂-NH₂/4-NMe₂SQ », « @Al₂O₃ b./4-NMe₂SQ », « @Al₂O₃ a./4-NMe₂SQ » à l'exposition à différents analytes NRBC-E**

| Particules | @SiO₂-NH₂ | | @Al₂O₃ b. | | @Al₂O₃ a. | |
|---|---|---|---|---|---|---|
| Eclairage | Lumière blanche | UV 365 nm | Lumière blanche | UV 365 nm | Lumière blanche | UV 365 nm |
| GA | - | Vert pâle | X | Bleui vert | orangé | Bleui vert |
| GB | rose | Bleu foncé | rouge | violet | Rose/ rouge | violet |
| VX | - | bleu clair | - | bleu clair | - | bleu clair |
| Novitchoks | - | turquoise intense | - | turquoise intense | jaune | turquoise intense |
| HD | rose | violet pâle | rouge | violet | rouge | violet fonce |
| HN3 | - | - | orange | violet | rouge | violet |
| L1 | blanc | bleu | blanc | bleu | blanc | bleu |
| C1 | rose | - | rose | bleu/ violet | blanc | bleu |
| C2 | - | - | rouge | rose | rose/ orange | - |

On observe que les signatures des toxiques déposés liquides (GA, GB, VX, Novitchoks, HD, HN3, L1, C1, C2) sont toutes différentes entre elles.

Parmi les plus semblables :
- L1 et C1 diffèrent par leur réponse sous lumière blanche sur le couple de détection « @Al₂O₃ b./4-NMe₂SQ ». En effet, L1 engendre une décoloration blanche, tandis que C1 provoque une coloration rose ;
- VX et Novitchoks diffèrent par leur réponse en fluorescence sous excitation à 365 nm sur les trois bandelettes utilisées dans cet exemple, ce qui fiabilise l'interprétation. En effet, le VX engendre une émission de fluorescence bleu clair, tandis que les Novitchoks engendrent une émission de fluorescence turquoise intense ;
- HD et HN3 diffèrent par leur réponse sous lumière blanche sur le substrat de détection « @SiO₂-NH₂/4-NMe₂SQ ». En effet, HD provoque une coloration rose, tandis que HN3 n'engendre aucun virage colorimétrique pour ce couple de détection ;
- GA et VX diffèrent par leur réponse sous lumière blanche sur le couple de détection « @Al₂O₃ a./4-NMe₂SQ », ainsi que par leur réponse en fluorescence sous excitation à 365 nm sur les trois couples de détection utilisés dans cet exemple. En effet, GA engendre une coloration orangée, tandis que le VX n'engendre aucun virage colorimétrique sur les trois couples de détection, De plus, GA engendre une émission de fluorescence vert pâle ou bleu/vert, tandis que le VX engendre une émission de fluorescence bleu clair.

## Revendications

1. Système de détection d'une ou plusieurs substances chimiques, comprenant au moins un composé de formule générale (I) : 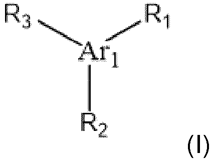 dans laquelle
Ar₁ représente un hétérocycle aromatique azoté éventuellement substitué par un ou plusieurs radicaux alkyle linéaires ou ramifiés,
ou Ar₁ représente un groupe aromatique polycyclique condensé contenant au moins un hétérocycle aromatique azoté, chacun des cycles dudit groupe aromatique polycyclique condensé étant carbocyclique ou azoté, et étant éventuellement substitué par un ou plusieurs radicaux alkyle linéaires ou ramifiés,
R₁, R₂ et R₃, identiques ou différents, représentent chacun un atome d'hydrogène ou un groupe R₄ de formule générale (II) : 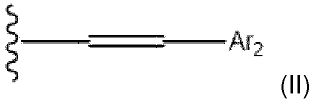 dans laquelle
Ar₂ représente un groupe phényl substitué par un groupement R₅ choisi parmi un atome d'hydrogène, un atome d'halogène, un groupe amino primaire, un groupe hydroxyle, un groupe sulfhydryle et un groupement -R₆, -NHR₆, -N(R₆)₂, -OR₆ ou -SR₆, où R₆ représente un radical alkyle linéaire ou ramifié, Ar₂ étant en outre éventuellement substitué par un ou plusieurs radicaux alkyle linéaires ou ramifiés,
R₁, R₂ et R₃ ne représentant pas tous simultanément un atome d'hydrogène, et dans lequel chaque composé de formule générale (I) particulier est porté par des particules d'au moins deux différents types, les particules d'un même type et portant un même composé de formule générale (I) étant disposées sur un support solide distinctement des autres.

2. Système selon la revendication 1, dans lequel, dans la formule générale (I), Ar₁ est choisi parmi les groupes de formules générales (IIIa), (IIIb), (IIIc) et (IIId) : 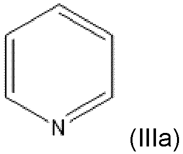 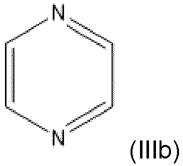 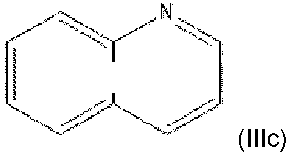 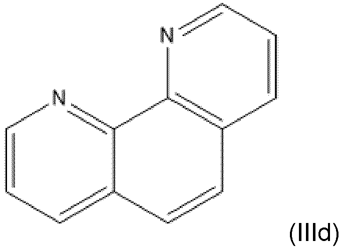 chacun desdits groupes de formules générales (IIIa), (IIIb), (IIIc) et (IIId) étant éventuellement substitué par un ou plusieurs radicaux alkyle linéaires ou ramifiés.

3. Système selon la revendication 1 ou 2, dans lequel, dans la formule générale (II), Ar₂ représente un groupe phényl substitué par ledit groupement R₅ en position para par rapport à la liaison vinylique à Ar₁.

4. Système selon l'une quelconque des revendications 1 à 3, dans lequel, dans la formule générale (I), R₂ et R₃ représentent un atome d'hydrogène, ou R₁ et R₂ sont identiques et R₃ représente un atome d'hydrogène, ou R₁, R₂ et R₃ sont identiques.

5. Système selon l'une quelconque des revendications 1 à 4, comprenant une pluralité de composés de formule générale (I) différents, chacun desdits composés étant porté par des particules d'au moins deux différents types.

6. Système selon l'une quelconque des revendications 1 à 5, dans lequel les particules d'au moins un desdits différents types de particules sont des particules inorganiques, de préférence choisies parmi les particules de silice, les particules de silice greffées par des groupes organiques, les particules d'alumine, les particules d'oxyde de zinc, les particules d'oxyde de titane, les particules de terre de diatomée, les particules de zéolithe, les particules en géopolymère.

7. Système selon l'une quelconque des revendications 1 à 6, dans lequel les particules sont disposées sur ledit support incluses dans une couche comprenant, outre ces particules, au moins un liant organique.

8. Système selon l'une quelconque des revendications 1 à 7, dans lequel ledit support est formé en papier.

9. Système selon l'une quelconque des revendications 1 à 8, dans lequel les particules d'un même type et portant un même composé de formule générale (I) sont disposées sur un support distinct.

10. Système selon l'une quelconque des revendications 1 à 8, dans lequel l'ensemble des particules sont disposées sur un même support, les particules d'un même type et portant un même composé de formule générale (I) étant disposées sur une zone distincte dudit support.

11. Procédé de préparation d'un système de détection selon l'une quelconque des revendications 1 à 10, comprenant des étapes de :
- préparation d'une pluralité de compositions différentes contenant chacune, respectivement, un composé de formule générale (I) particulier porté par des particules d'un différent type de particules, au moins un solvant organique, et éventuellement au moins un liant organique,
- et dépôt de chacune desdites compositions sur un support solide, distinctement les unes des autres.

12. Procédé de préparation selon la revendication 11, selon lequel le ou les solvants organiques sont des solvants apolaires.

13. Procédé de préparation selon la revendication 11 ou 12, selon lequel le ou les solvants organiques sont des solvants hydrocarbures choisis parmi les solvants aliphatiques saturés, les solvants aromatiques et les solvants terpénoïdes.

14. Procédé de préparation selon l'une quelconque des revendications 11 à 13, selon lequel le ou les liants organiques sont des liants polymériques, de préférence comprenant des unités répétitives styréniques, de type polydiméthylsiloxane, ou de type paraffine.

15. Procédé de préparation selon l'une quelconque des revendications 11 à 14, selon lequel le dépôt desdites compositions est réalisé respectivement sur des supports distincts.

16. Procédé de préparation selon l'une quelconque des revendications 11 à 14 selon lequel le dépôt desdites compositions est réalisé respectivement sur des zones distinctes d'un même support.

17. Procédé de détection d'une substance chimique susceptible d'être contenue dans un milieu, comprenant des étapes de :
- mise en contact dudit milieu avec l'ensemble des particules portant un composé de formule générale (I) d'un système de détection selon l'une quelconque des revendications 1 à 10 ;
- acquisition de la réponse colorimétrique et de la réponse fluorogénique induites par ladite mise en contact, pour chaque couple « particules d'un même type / même composé de formule générale (I) » différent compris dans ledit système de détection,
- déduction, en fonction de l'ensemble desdites réponses colorimétriques et desdites réponses fluorogéniques, de l'absence ou de la présence de ladite substance dans ledit milieu.

18. Procédé selon la revendication 17, selon lequel ladite substance chimique est un composé toxique de guerre, un composé toxique industriel chimique et/ou un pesticide.

19. Procédé selon la revendication 17 ou 18, selon lequel ladite substance chimique est une substance organophosphorée.

20. Kit de détection d'une ou plusieurs substances chimiques, comportant :
- un système de détection selon l'une quelconque des revendications 1 à 10,
- une base de données colorimétriques et fluorogéniques permettant de faire la correspondance entre les réponses colorimétriques et les réponses fluorogéniques observées pour chaque couple « particules d'un même type / même composé de formule générale (I) » différent compris dans ledit système de détection, et chacune desdites substances chimiques.
